# EUROPEAN PATENT SPECIFICATION

(11) **EP 1 644 395 B1**
(45) Date of publication and mention of the grant of the patent: **22.11.2006**
(21) Application number: 04739843.3
(22) Date of filing: 11.06.2004
(51) Int. Cl.: C07H 19/067

(54) **PROCESSES FOR PREPARING 4'AZIDO NUCLEOSIDE DERIVATIVES**
VERFAHREN ZUR HERSTELLUNG VON 4'-AZIDONUKLEOSIDDERIVATEN
METHODES DE PREPARATION DE DERIVES DE 4'AZIDO NUCLEOSIDE

(30) Priority: 19.06.2003 US 479796 P
(43) Date of publication of application: 12.04.2006
(73) Proprietor: F. HOFFMANN-LA ROCHE AG, 4070 Basel (CH)
(72) Inventor: CONNOLLY, Terrence, Joseph, Redwood City, CA 94061 (US); DURKIN, Kieran, Folsom, CA 95630 (US); SARMA, Keshab, Sunnyvale, CA 94087 (US); ZHU, Jiang, Cupertino, CA 95014 (US)
(74) Representative: Rauber, Beat
(86) International application number: PCT/EP2004/006357
(87) International publication number: WO 2005/000864

(56) References cited:
- WO-A-02/100415
- MAAG H ET AL: "Synthesis and anti-HIV activity of 4'-azido- and 4'-methoxynucleosides" JOURNAL OF MEDICINAL CHEMISTRY, AMERICAN CHEMICAL SOCIETY. WASHINGTON, US, vol. 35, 1992, pages 1440-1451, XP002277180 ISSN: 0022-2623 cited in the application

## Description

The invention relates to a novel process to prepare 4'-azidonucleoside derivatives that are useful for treating virus mediated diseases. More specifically the invention relates to a process for preparing 4-amino-1-((2R,3R,4S,5R)-5-azido-3,4-dihydroxy-5-hydroxymethyl-tetrahydro-furan-2-yl)-1H-pyrimidin-2-one and pharmaceutically acceptable acid addition salts thereof and more particularly the hemisulfate salt.

The invention relates to processes for the preparation of azido substituted nucleoside derivatives useful for treating viral-mediated diseases. In particular, the invention is concerned with processes to prepare 4-azido pyrimidine nucleoside derivatives which are useful inhibitors of Hepatitis C Virus (HCV) RNA replication.

Hepatitis C virus (HCV) is responsible for a large proportion of the chronic liver disease worldwide and accounts for 70% of cases of chronic hepatitis in industrialized countries. The global proportion of hepatitis C is estimated to average 3% (ranging from 0.1% to 5.0%) and there are an estimated 170 million chronic carriers throughout the world. There is a continuing need for effective therapeutic agents against HCV and processes for the manufacture of such agents.

J. G. Moffatt *(Chemical Transformations of the Sugar Moiety of Nucleosides,* in *Nucleoside Analogues,* R. T. Walker, E. De Clercq and F. Eckstein, eds., Plenum Publishing Corp., New York, 1979, p. 144) describe the preparation of 4'-azidocytidine (VIIIb) by electrophilic addition of iodine azide to N-[1-((2R,3R,4S)-3,4-dihydroxy-5-methylene-tetrahydro-furan-2-yl)-2-oxo-1,2-dihydro-pyrimidin-4-yl]-benzamide (X).

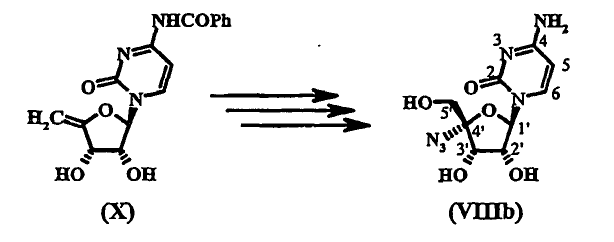

Maag *et al.* (*J. Med. Chem.* 1992 35:1440-1451) disclose the preparation of 4'-azido nucleosides by addition of iodine azide to 5-methylene-tetrahydro-furan-2-yl nucleosides XI wherein B is 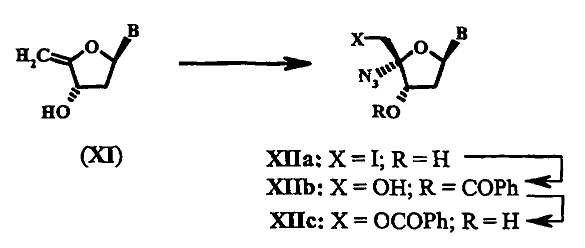 thymine, uracil, adenine or guanosine. Maag *et al.* (*supra*) further disclose that although displacement of the 5'-iodo is retarded by electron-withdrawing groups at the 4'-position, contacting (XIIa) with perbenzoic acid derivatives oxidize the iodide to a hypervalent state which is then displaced to afford a mixture of products including XIIb and XIIc. The reaction was suggested to proceed *via* a 3'-5'-cyclic benzoxonium ion. The criticality of the proximal 3'-acyloxy moiety was apparent when the reaction failed with a 3'-deoxy-nucleoside or a nucleoside unesterified at the 3'-position.

WO 02/100415 (R. Devos *et al.*) discloses 4'-substituted nucleoside derivatives which inhibit viral DNA polymerase. 4'-Azidonucleosides were prepared by the method described by Maag *et al.* (*supra*)*.* The acetonide of uridine IVd was iodinated using a mixture of TPP, iodine and pyridine to afford the corresponding 5'-iodo derivative IVe. The acetonide protecting group was removed by treatment with an acid, for instance acetic acid, as described by J. P. Verheyden *et al.* (*J. Org. Chem.,* 1970, 35(7): 2319) to afford nucleosides of formula IVf which were dehydroiodinated with sodium methoxide to afford Va. Treatment of Va with a mixture of iodine chloride and sodium azide in DMF afforded iodoazides nucleosides of formula IIa. After the hydroxy groups of IIa were protected by treatment of with benzoyl chloride in pyridine to afford diacyl nucleosides of formula IId, the diesters were converted into the 5'-benzoyl nucleosides of formula IIIb by treatment with MCPBA in dichloromethane. The uridine IIIb was converted to cytidine by protecting the 3'-hydroxy with Ac₂O and pyridine and utilizing the method described by A. D. Borthwick *et al.,* (*J. Med. Chem.* 1990, 33(1):179; see also K. J. Divakar and C. B. Reese *J. Chem Soc., Perkin Trans. I* 19821171-1176). Thus IIIc was treated with 4-chlorophenyl dichlorophosphate and 1,2,4-triazole to give 4-triazolyl nucleosides of formula XIII, which was displaced with aqueous ammonia giving 4'-substituted cytidines of formula VIII.

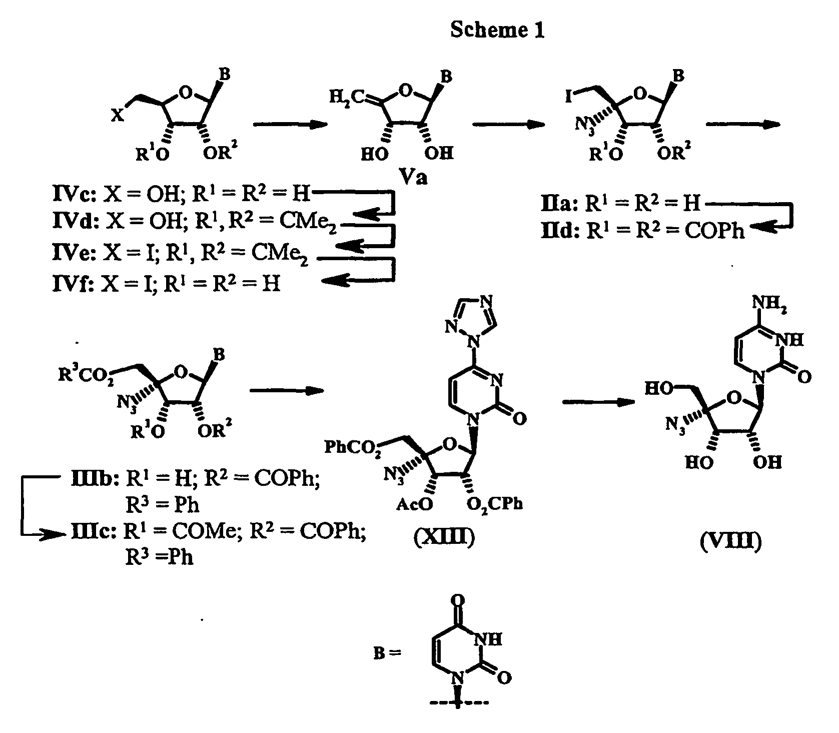

The present invention further relates to processes to prepare 4'-azido-uridine derivatives (I) or 4'-azido-cytidine (VIII) or acid addition salts thereof.

4'-Azidocytidine has now been found to possess excellent activity against HCV polymerase. There is a need for new efficient processes to prepare VIII, and acid addition salts thereof, that minimizes process steps and reduces dependence on inefficient protecting strategies. Further the process steps must be compatible with a thermolabile azide moiety. The present invention relates to novel processes to prepare 4'-azidonucleoside compounds and 4'-azido-2',3',5'-tri-acylnucleoside compounds.

One object of present invention is (i) process for the preparation of a 4'-azido-cytidine compound of formula VIII 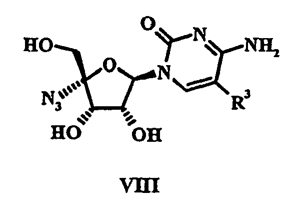 and pharmaceutically acceptable addition salts thereof
wherein
- R³: is H, C₁₋₆-alkyl, C₁₋₃-haloalkyl or halogen
characterized in that
in step a)
   nucleoside compound of formula VIa 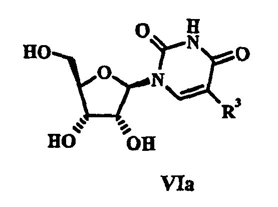 is transformed to a compound of formula II 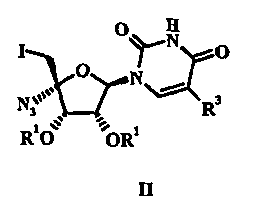 wherein
   - R¹: is R^{1a}CO-;
   - R^{1a}: is C₁₋₁₀-alkyl or phenyl optionally substituted with 1 to 3 substituents selected from the group consisting of alkyl, alkoxy, halogen, nitro or cyano;
   - R³: is as defined above,
in step b)
   a solution of 5'-iodo compound of formula II in a suitable solvent is contacted with a peracid, R^{2a}C(O)OOH, an acid R^{2a}C(O)OH and optionally a phase transfer catalyst to produce a 4'-azido-2',3',5'-triacyl-nucleoside compound of formula I 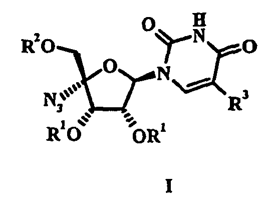 wherein
   - R¹: is R^{1a}CO-;
   - R²: is R^{2a}CO-;
   - R^{1a} and R^{2a}: are independently C₁₋₁₀-alkyl or phenyl optionally substituted with 1 to 3 substituents selected from the group consisting of alkyl, alkoxy, halogen, nitro or cyano
   - R³: is as defined above,
in step c)
   the 4'-azido-2',3',5'-triacyl-nucleoside compound of formula I is transformed to afford a 4'-azido-cytidine compound of formula VIII and if necessary, a pharmaceutically acceptable addition salts.

Further objects of the present invention are:
(ii) Process for preparation of a 4'-azido-2',3',5'-triacyl-nucleoside of formula I, comprising contacting a a solution of 5'-iodo compound of formula II in a suitable solvent is contacted with a peracid, R^{2a}C(O)OOH, an acid R^{2a}C(O)OH and optionally a phase transfer catalyst to produce a 4'-azido-2',3',5'-triacyl-nucleoside compound of formula I 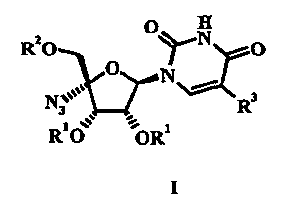 wherein
   - R¹: is R^{1a}CO-;
   - R²: is R^{2a}CO-;
   - R^{1a} and R^{2a}: are independently C₁₋₁₀-alkyl or phenyl optionally substituted with 1 to 3 substituents selected from the group consisting of alkyl, alkoxy, halogen, nitro or cyano;
   - R³: is H, C₁₋₆-alkyl, C₁₋₃-haloalkyl or halogen.
(iii) Process according to (i) for the preparation of the 4'-azido-2',3',5'-triacyl-nucleoside compound of formula Ia, characterized in that said process further comprises contacting said 4'-azido-2',3',5'-triacyl-nucleoside compound of formula IV with a first base to afford a 4'-azido-nucleoside compound of formula Ia 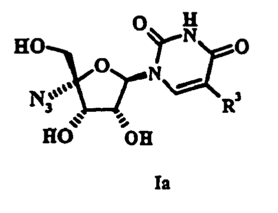 wherein R³ is as defined in (i).
(iv) Process according to (i) for preparing a 5'-iodo compound of formula II, characterized in that said process comprises the steps of
   a) contacting nucleoside IVa with an halogenating agent to produce a 5'-halo nucleoside compound of formula IVb 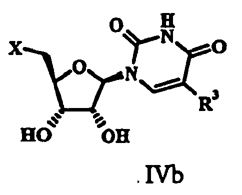 wherein X is a halogen, R³ is as defined in (i);
   b) contacting compound of formula IVb with a dehydrohalogenating agent to produce a 5-methylene nucleoside compound Va 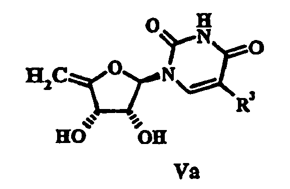 wherein R³ is as defined above;
   c) contacting compound of formula Va with a quaternary ammonium azide and iodine dissolved in tetrahydrofuran(THF) and acetonitrile(MeCN) to produce an iodo azide compound of formula IIa 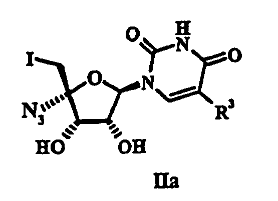 wherein R³ is as defined above;
   d) contacting compound of formula IIa with at least one second base and a first acylating agent to afford diester compound of formula II 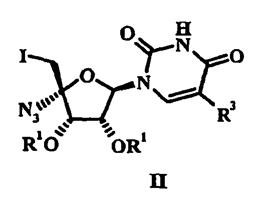
   wherein R¹ and R³ are as defined above.
(v) Process for preparing 4'-azidocytidine compound of formula VIII according to (i), characterized in that said process comprises the steps of:
   a) contacting compound of formula I 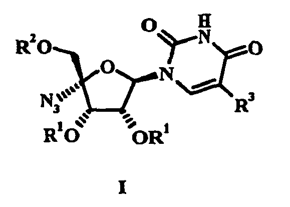 wherein R1 and R2 are defined in (i), R³ is hydrogen with 1,2,4-triazole, phosphorus oxychloride, triethylamine(TEA) in CH₂Cl₂ to afford triazole compound of formula VI 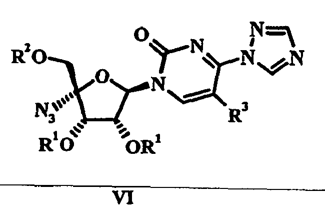
   b) contacting compound of formula VI with a solution of ammonium hydroxide and tetrahydrofuran(THF) to displace the triazole to afford a 4'-azido-2',3',5'-triacylcytidine compound of formula VII 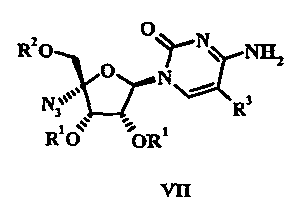
   c) contacting compound of formula VII with a solution of ammonia and an alcohol to cleave the esters to afford compound of formula VIII 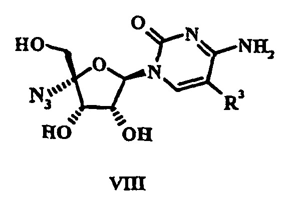
(vi) Process according to (iv), characterized in that said process comprises the steps of:
   a) contacting compound of formula Va 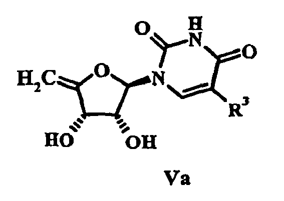 with a second acylating agent and optionally with a trialkylamine base to afford diacyl compound Vb 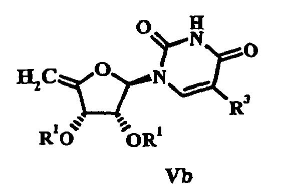 wherein R¹ and R³ are as defined in (iv),
      and washing an organic solution of Vb with aqueous NaHCO₃ wherein the pH was maintained at about 7.5; and,
   b) contacting said diacyl compound of formula Vb with a solution of ammonia and methanol to regenerate Va.
(vii) Process according to (i) for preparing the hemisulfate acid addition salt of 4'-azidocytidine of formula VIIIa 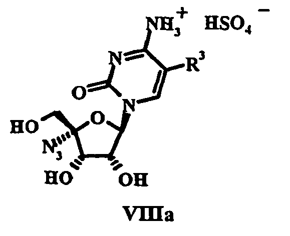 wherein R3 is as defined in (i);
   characterized in that said process further comprises the crystallizing a compound of formula VIII from isopropanol, water and sulfuric acid.
(viii) Process according to (vii) for the preparation of the hemisulfate acid addition salt of 4'-azido-cytidine of formula VIIIa wherein R³ is hydrogen; R¹ is PhCO; R² is R^{2a}CO
   wherein R^{2a} is optionally substituted phenyl; said phase transfer catalyst is a tetraalkyl ammonium hydrogen sulfate, said solvent is mixture of an aqueous buffer and a nonpolar organic solvent, said iodinating agent is triphenylphosphine(TPP), iodine and imidazole; said dehydrohalogenating agent is sodium methoxide or 1,8-diazabicyclo[4.3.0]non-5-ene(DBN); said quaternary ammonium azide is benzyl triethylammonium azide and said acylating agent is benzoyl chloride, said first base is ammonia and said second base is a N-methylinorpholine(NMM)and 4-dimethylaminopyridine(DMAP) and said alcohol is methanol.
(ix) Process for the preparation of 4'-azidorytidine hemisulfate of formula VIIIc 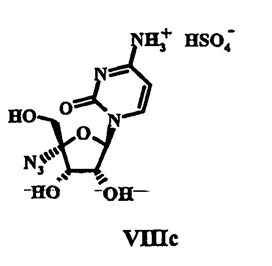 characterized in that said process comprises the steps of:
   a) contacting a tetrahydrofuran(THF) solution of uridine compound of formula IVg 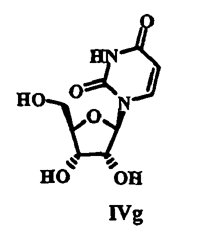 with triphenylphosphine(TPP), iodine and imidazole to produce 1-(3,4-dihydroxy-5-halomethyl-tetrahydro-furan-2-yl)-1H-pyrimidine-2,4-dione of formula IVh 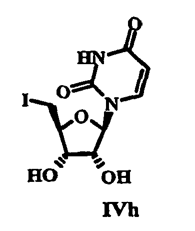
   b) contacting compound of formula IVh with a methanolic solution of sodium methoxide agent to produce 1-(3,4-dihydroxy-5-methylene-tetrahydro-furan-2-yl)-1H-pyrimidine-2,4-dione of formula Vc; 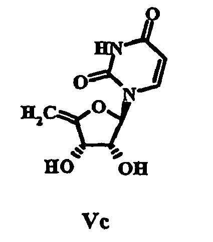
   c) contacting compound of formula Vc with a solution of benzyl triethylammonium azide and iodine in tetrahydrofuran(THF) and acetonitrile(MeCN) to afford iodo azide of formula IIe; 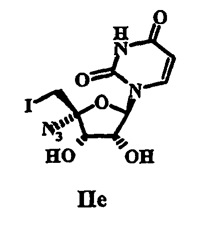
   d) contacting compound of formula IIe with a solution benzoyl chloride, N-methylmorpholine(NMM) and acetonitrile(MeCN) to afford the dibenzoate compound of formula IIg: 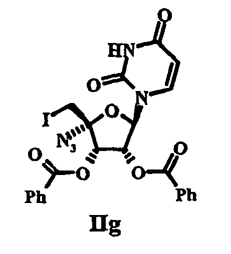
   e) contacting compound of formula IIg with *m*-chloroperbenzoic acid, *m*-chlorobenzoic acid and tetrabutylammonium hydrogen sulfate in a two-phase solution of dichloromethane(DCM) and an aqueous solution of potassium hydrogen phosphate to afford compound of formula IIId; 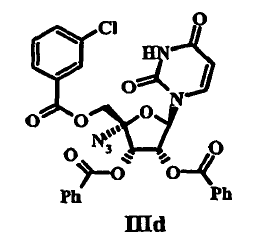
   f) contacting compound of formula IIId with a solution of 1,2,4-triazole, phosphorus oxychloride, triethylamine(TEA) in dichloromethane(DCM) to afford compound of formula IIIe; 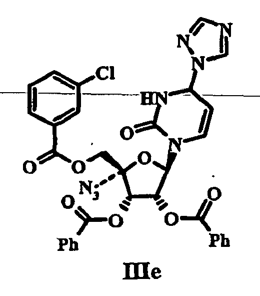
   g) contacting compound of formula IIIe with methanol solution of ammonia to cleave the esters and afford free base of compound of formula VIIIb; 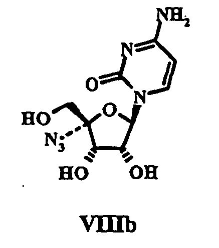
   h) crystallize compound of formula VIIIb from isopropanol and water containing H₂SO₄ to afford the hemisulfate salt of formula VIIIc. 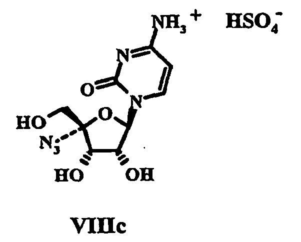
(x) A compound according to formula Ic 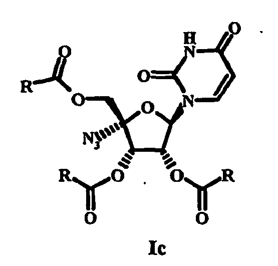
wherein R is phenyl optionally substituted with one to three substituents selected from the group consisting of halogen, C₁₋₁₀-alkyl, C₁₋₁₀-alkoxy, nitro, cyano.

In one embodiment of the present invention there is provided a process for the preparation of a 4'-azido-2',3',5'-triacyl-nucleoside compound according to formula I, 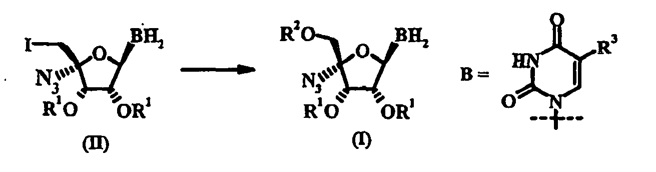 wherein R¹ is R^{1a}CO-, R² is R^{2a}CO-, R^{1a} and R^{2a} are independently C₁₋₁₀ alkyl or phenyl optionally substituted with 1 to 3 substituents selected from the group consisting of alkyl, alkoxy, halogen, nitro or cyano; R³ is selected from the group consisting of hydrogen, C₁₋₆ alkyl, C₁₋₃ haloalkyl and halogen , said process comprising contacting a solution of a 5'-iodomethyl-2',3'-diacyl nucleoside compound II in a suitable solvent with a carboxylic peracid, R^{2a}C(O)OOH, a carboxylic acid R^{2a}C(O)OH and optionally a phase transfer catalyst.

Surprisingly the displacement of the iodo-dibenzoate (II) by activation of the leaving group with an oxidizing agent (MCPBA), and displacement with an external nucleophile (MCBA) in a suitable solvent containing a phase-transfer catalyst resulted in significantly higher yields of a triester then the displacement reaction described by Maag *et al.* (*supra*). The reaction directly affords a triester and eliminates the need for a separate reaction step to esterify the 3'-hydroxyl group formed in the absence of an external nucleophile. To activate the leaving group the iodide is oxidized to a hypervalent state with a carboxylic peracid. The peracid activation is necessitated by the presence of an electron-withdrawing group at the 4'-position. Previous studies have shown that the labile hypervalent iodide was displaced by an intramolecular transfer of the acyl moiety on the 3'-hydroxyl to the 5'-hydroxyl. The present reaction, in contrast, proceeds via an intermolecular displacement by carboxylic acid salt (e.g., potassium *m-*chlorobenzoate, sodium benzoate, sodium acetate) rather than intramolecular transfer of the 3-acyl moiety. The reaction can be carried out in a variety of solvents. Any organic solvent which is sufficient polar to dissolve the salt of the nucleophilic carboxylic acid is acceptable; however, a two-phase media including a buffered aqueous solution to provide nucleophilic carboxylic acid salt, a phase-transfer catalyst and a non-polar organic solvent is especially suitable. A two-phase media comprising water and dichloromethane is preferred due to non-flammability of the organic solvent. Suitable buffers afford a pH from about 2 to about 10 and include, but are not limited to potassium phosphate, potassium hydrogen phosphate, dihydrogen potassium, sodium carbonate and sodium bicarbonate. The present process allows for the introduction a specific 5-acyloxy moiety in good yield and high purity.

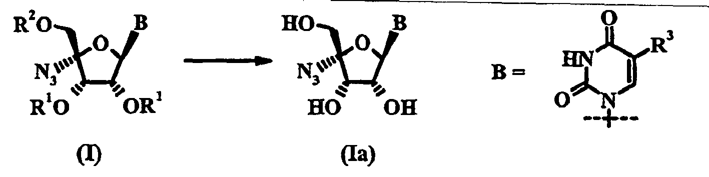

In another embodiment of the present invention there is provided a process for the preparation of a 4'-azido-nucleoside compound Ia wherein R³ is as defined hereinabove, said process comprising (*i*) contacting a solution of a 5'-iodomethyl-2',3'-diacyl nucleoside compound II in a suitable solvent with a carboxylic peracid, R^{2a}C(O)OOH, a carboxylic acid R^{2a}C(O)OH and optionally a phase transfer catalyst to afford I wherein R¹, R² and R³ are as described hereinabove; and (*ii*) contacting the resulting 4'-azido-2',3',5'-triacyl-nucleoside compound I with a base to afford the pyrimidine nucleoside compound according to formula Ia affording a novel and efficient process for the to prepare 4'-azido pyrimidine nucleosides.

In another embodiment of the present invention there is provided a method for the preparation of 4'-azidopurine derivatives of formula Ia wherein B is hypoxanthine, adenine or guanine wherein the 6-amino of adenine or the 2-amino of guanine is masked by an N-protecting group until the azido moiety is incorporated.

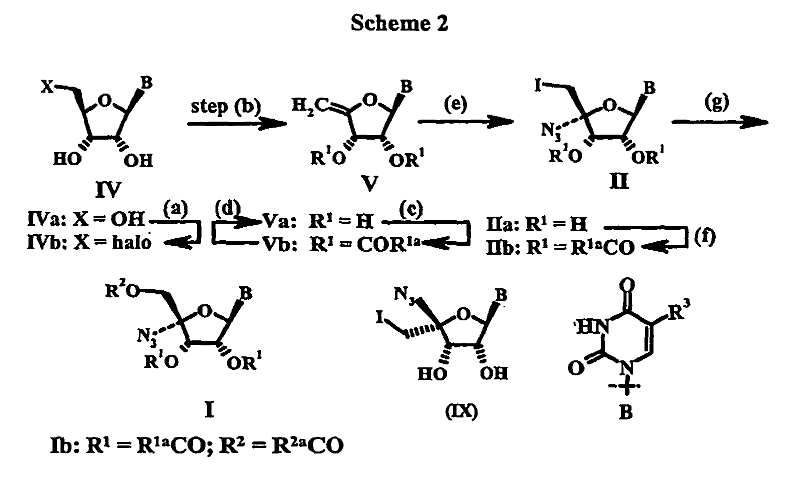

In another embodiment of the present invention there is provided a process (Scheme 2) for the preparation of a 4'- azido-2',3',5'-triacyl-nucleoside compound according to formula I, wherein R¹, R² and R³ are as defined hereinabove from a nucleoside IVa said process comprising (*i*) contacting IVa with a halogenating agent to afford IVb ; (*ii*) contacting IVb with a dehydrohalogenating agent to produce a 5-methylene nucleoside compound Va; (*iii*) contacting Va with a quaternary ammonium azide and iodine dissolved in a polar aprotic solvent to produce iodo azide IIa; (*iv*) contacting IIa with an acylating agent to afford diester IIb; and, (*v*) contacting a solution of a 5'-iodomethyl-2',3'-diacyl nucleoside compound IIb in a suitable solvent with a carboxylic peracid, R^{2a}C(O)OOH, a carboxylic acid R^{2a}C(O)OH and optionally a phase transfer catalyst to afford I.

Elimination of protection/deprotection steps is generally desirable to minimize the number of discrete steps in a process. T. Tsuji and K. Takenaka (*Nucleosides & Nucleotides* 1987 6(3):575-80) disclose the bromination or iodination of the 5'-hydroxymethyl of unprotected nucleosides with carbon tetrahalides and TPP in DMF or HMPA. Maag *et al. (supra)* disclose a procedure for selective iodination of unprotected nucleosides by contacting a dioxane solution of the nucleoside with iodine and TPP in the presence of pyridine or imidazole. Yields of 29 to 59% were reported. Other methods for converting alcohols to iodides are well known (see, *e.g.,* J. March, *Advanced Organic Chemistry,* John Wiley & Sons, 4^{th} edition, 1992, pp. 431-433). In the step (a) of the present process the reaction conditions have been improved and optimized by replacing dioxane with THF and carefully controlling the temperature of the reaction resulting in a yield of 84% of IVb.

Dehydrohalogenation of 5'-halonucleosides has been described (T. Ueda, in *Chemistry of Nucleotides and Nucleosides,* L. B. Townsend (ed.), Plenum Press, NY, 1988, pp. 83-88). Other examples of dehydrohalogenation reagents are described by March and references cited therein (J. March, *supra* pp.1023-1025). Maag *et al.* (*supra*) disclosed the dehydroiodination of related nucleoside derivatives with DBN or sodium methoxide. In the step (b) of the present process dehydrogenation the sodium methoxide provided the best results. Excess sodium methoxide was quenched by addition of N-methylmorpholinium mesylate.

Step (e) of the present process is a formal electrophilic addition of iodine azide to the exocyclic olefin. In view of the known explosion and toxic hazards of organo azides (see, *e.g.,* M.E.C. Biffin *et al.* in *Chemistry of the Azido Group,* S. Patai (ed.) Wiley-Interscience, New York, 1971, pp 61-63) and iodine azide (N. I. Sax and R.J. Lewis, Sr., *Dangerous Properties of Industrial Materials,* van Nostrand, New York 1989, p. 1993) synthetic reactions with organoazides must be carefully designed to minimize hazards as well as to increase efficiency. This is particularly true when the goal is a commercial scale manufacturing. Maag *et al. (supra)* and Moffatt *(supra)* both disclose the addition of iodine azide to 5-methylene nucleosides. Surprisingly it has now been found in differential scanning calorimetry and ARSST [Advanced Reaction System Screening Tool] that mixtures quaternary ammonium azides and iodine are less prone to violent decomposition then is iodine azide and efficiently add to 5-methylene nucleosides to afford iodo azides. Preferably the quaternary ammonium salt is a phase transfer catalyst and especially benzyl triethylammonium azide. Thus benzyl triethyl ammonium chloride and sodium azide are slurried in acetonitrile and the solution of benzyl triethylammonium azide (BTEAA) is filtered from the precipitated sodium chloride. A MeCN solution of the BTEAA, Va and NMM is cooled to 0-5° C and a solution of iodine and THF was added to produce the desired iodo azide IIa and its diastereomer IX in a ratio of at least about 9:1 (IIa:IX). Excess azide is eliminated by addition of a small quantity of N-acetylrysteine which catalyzes oxidation of azide by iodine and the reaction product is then converted to the dibenzoate IIb (R^{1a} = Ph) *in situ* by addition of at least one base and benzoyl chloride while maintaining an internal temperature at *ca.* 5° C. The dibenzoate thus obtained is subjected to the percarboxylate/carboxylate (*e.g.,* MCPBA/MCBA) mediated displacement to afford I.

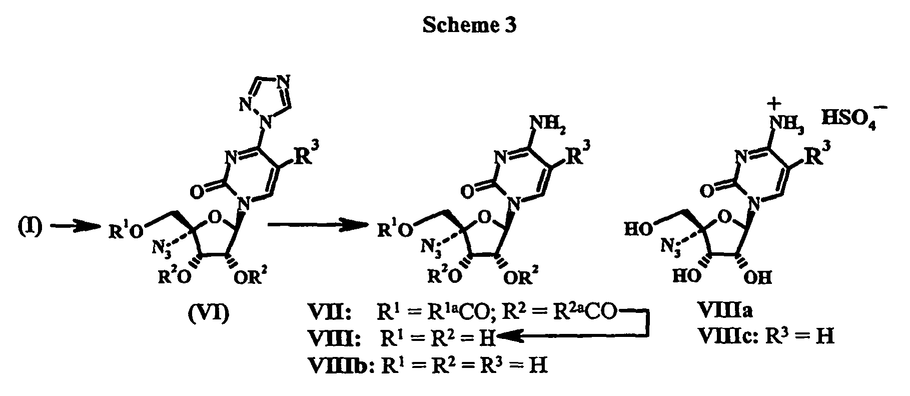

In another embodiment of the present invention there is provided a process (Scheme 2 & 3) for preparing 4'-azido-cytidine VIII wherein R³ is as defined hereinabove, said process comprising the steps (*i*) contacting nucleoside IVa with an halogenating agent to produce a 5'-halo nucleoside compound IVb; (*ii*) contacting IVb with a dehydrohalogenating agent to produce a 5-methylene nucleoside compound Va; (*iii*) contacting Va with a quaternary ammonium azide and iodine dissolved in an aprotic polar media to produce a iodo azide IIa; (*iv*) contacting IIa with an acylating agent to afford diester IIb wherein R¹, R² and R³ are as described hereinabove and (*v*) contacting a solution of a 5'-iodomethyl-2',3'-diacyl nucleoside compound II in a suitable solvent with a carboxylic peracid, R^{2a}C(O)OOH, a carboxylic acid R^{2a}C(O)OH and optionally a phase transfer catalyst to afford I; (vi) contacting I with 1,2,4-triazole, phosphorus oxychloride and TEA in CH₂Cl₂ to afford triazole VI; (*vii*) contacting VI with a solution of ammonium hydroxide in an aprotic solvent (e.g., MeCN, THF or DMF) to displace the triazole to afford a 4'-azido-2',3',5'-triacylcytidine VII; (*viii*) contacting VII with a solution of ammonia and an alcohol to cleave the esters to afford VIII. The two step ammonolysis affords products of sufficient purity that no column chromatography is required to afford a final product with acceptable purity.

In another embodiment of the present invention there is provided a process (Scheme 2) for the preparation of a 4'-azido-2',3',5'-triacyl-nucleoside compound according to formula I, wherein R¹, R², and R³ are as defined hereinabove from nucleoside IVa said process comprising (*i*) contacting IVa with an halogenating agent to produce a 5'-halo nucleoside compound IVb wherein R¹ is hydrogen; (*ii*) contacting IVb with a dehydrohalogenating agent to produce a 5-methylene nucleoside compound Va; (*iii*) treating Va with an acylating agent to afford Vb, extracting a solution of Vb and a water immiscible solvent with aqueous NaHCO₃ with a pH at least about 7.5; (*iv*) removing the acyl groups to regenerate Va; (*v*) contacting Va with a quaternary ammonium azide and iodine dissolved in THF and MeCN to produce a iodo azide IIa; (vi) contacting IIa with an acylating agent to afford diester IIb; and (*vii*) contacting a solution of a 5'-iodomethyl-2',3'-diacyl nucleoside compound II in a suitable solvent with a carboxylic peracid, R^{2a}C(O)OOH, a carboxylic acid R^{2a}C(O)OH and optionally a phase transfer catalyst to afford I; (*viii*) contacting I with 1,2,4-triazole, phosphorus oxychloride and TEA in CH₂Cl₂ to afford triazole VI; (*ix*) contacting VI with a solution of ammonium hydroxide and in a polar aprotic solvent to displace the triazole to afford a 4'-azido-2',3',5'-triacylcytidine VII; (*x*) contacting VII with a solution of ammonia and an alcohol to cleave the esters to afford VIII..

In another embodiment of the present invention there is provided a process for preparing the hemisulfate acid addition salt of 4'-azidorytidine VIIIa said process comprising the preparation of VIII as described in Schemes 2 & 3 and further comprising the steps of adding sulfuric acid to the isopropanol/water mixture used to crystallize VIII.

Purification by column chromatography is a common laboratory technique but it is expensive and laborious in larger scale processes. In addition, chromatography produces large volume of solvents which are costly and have to be discarded or recycled. Thus modifications that eliminate chromatography are advantageous. Steps (*iii*) and (*iv*) in the previous embodiment can optionally be incorporated into the process to add an aqueous base wash if further purification of the 5-methylene nucleoside intermediate Va is required. Furthermore the dehydroiodination, acylation, washing and deacylation can be carried out in a single vessel.

In another embodiment of the present invention there is provided a process (Scheme 2 & 3) for preparing 4'-azido-cytidine VIIIa wherein R³ is hydrogen comprising the steps (*i*) contacting nucleoside IVa with TPP, iodine and imidazole to produce a 5'-iodo nucleoside compound IVb (X = I) wherein R¹ is hydrogen; (*ii*) contacting IVb (X = I) with DBN or sodium methoxide to produce a 5-methylene nucleoside compound Va; (iii) contacting Va with a benzyl triethylammonium azide and iodine dissolved in THF and MeCN to produce a iodo azide IIa; (*iv*) contacting IIa with benzoyl chloride to afford the diester IIb (R¹ = PhCO); and (*v*) contacting a solution of a 5'-iodomethyl-2',3'-diacyl nucleoside compound IIb (R¹ = PhCO) in an aqueous buffer and a nonpolar organic solvent with a carboxylic peracid, R^{2a}C(O)OOH, a carboxylic acid R^{2a}C(O)OH and a phase transfer catalyst to afford Ib (R^{1a} is Ph and R^{2a} is optionally substituted Ph); (*vi*) contacting Ib (R^{1a} is Ph, R^{2a} is optionally substituted Ph and R³ is hydrogen) with 1,2,4-triazole, phosphorus oxychloride and TEA in CH₂Cl₂ to afford triazole VI (R^{1a} is Ph, R^{2a} is optionally substituted Ph and R³ is hydrogen); (vii) contacting VI (R^{1a} is Ph, R^{2a} is optionally substituted Ph and R³ is hydrogen) with a solution of ammonium hydroxide and acetonitrile to displace the triazole and afford a 4'azido-2',3',5'-triacylcytidine VII (R^{1a} is Ph, R^{2a} is optionally substituted Ph and R³ is hydrogen); (*viii*) contacting VII (R^{1a} is Ph, R^{2a} is optionally substituted Ph and R³ is hydrogen) with a solution of ammonium hydroxide and an alcohol to cleave the esters to afford VIIIb which crystallized from isopropanol/water/ sulfuric acid to afford the hemisulfate salt VIIIc.

Moffat *(supra)* disclosed the addition of iodine azide to 5'-methylene N-benzoyl cytidine (X). It has now been found that the over all process is more efficient when the nucleoside base is a 1H-pyrimidine-2,4-dione. The present embodiment is capable of producing uridine and cytidine nucleosides by inter-converting the bases linked to the nucleoside. In the present process there is provided a method to convert a uridine or thymine to a cytidine. The conversion of thymine to uridine by addition of triazoles has been described by Maag *(supra),* A. D. Borthwick *(supra)* and Divakar and Reese *(supra).* Displacement of the triazole with ammonia and cleavage of the triesters were best accomplished selectively by sequentially reacting VI (R¹ is R^{1a}CO and R² is R^{2a}CO and R³ is hydrogen) with ammonium hydroxide in a polar aprotic solvent which produced cytidine (VII) and reacting the triester VII with ammonia and methanol at about 45° C which cleaved the benzoyl esters to afford VIII along with methyl benzoate and benzamide.

In another embodiment of the present invention there is provided a process (Scheme 2 & 3) for preparing the hemisulfate salt of 4'-azido-cytidine VIIIc wherein R¹ is PhCO, X is *m*-chloro-benzoyloxy, R² is *m*-chlorobenzoyl R^{2a} is *m*-chlorophenyl, and R³ is hydrogen said process comprising the steps (*i*) contacting nucleoside IVg with TPP, iodine and imidazole to produce a 5'-iodo nucleoside compound IVh; (*ii*) contacting IVh with a methanol solution of sodium methoxide to produce a 5-methylene nucleoside compound Vc; (*iii*) contacting Vc with a benzyl triethylammonium azide and iodine dissolved in THF and MeCN to produce a iodo azide IIe; (*iv*) contacting IIe with benzoyl chloride to afford diester IIg; (*v*) contacting a 5'-iodomethyl-2',3'-diacyl nucleoside compound IIg with MCPBA, MCBA, tetrabutylammonium hemisulfate in a two-phase medium composed of DCM and aqueous potassium hydrogen phosphate (2 molar equivalents of K₂HPO₄ relative to MCPBA) to afford IIId; (*vi*) contacting IIId with 1,2,4-triazole, phosphorus oxychloride and TEA in CH₂Cl₂ to afford triazole IIIe; (*vii*) contacting IIIe with a solution of ammonium hydroxide and in a aprotic polar solvent to displace the triazole and afford a 4'azido-2',3',5'-triarylcytidine VII (R^{1a} is Ph, R^{2a} is 3-Cl-Ph, R³ is hydrogen); (*viii*) contacting VII (R^{1a} is Ph, R^{2a} is 3-Cl-Ph, R³ is hydrogen) with a solution of ammonia and an methanol to cleave the esters to afford VIIIb which crystallized from isopropanol/water/ sulfuric acid after removal of the MeOH to afford the hemisulfate salt VIIIc.

In another embodiment of the present invention there is provided novel compounds according to formula Ic 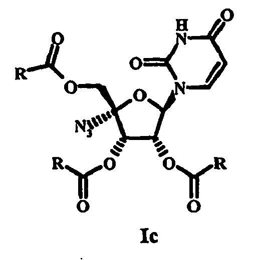 wherein R is phenyl optionally substituted with one to three substituents selected from the group consisting of halogen, C₁₋₁₀ alkyl, C₁₋₁₀ alkoxy, nitro, cyano which are useful intermediates in the preparation of VIIIc.

Unless otherwise stated, the following terms used in this Application, including the specification and claims, have the definitions given below. The phrase "a" or "an" entity as used herein refers to one or more of that entity; for example, a compound refers to one or more compounds or at least one compound. As such, the terms "a" (or "an"), "one or more", and "at least one" can be used interchangeably herein.

In general, the systematic nomenclature used in this Application is based on AUTONOM^{™} v.4.0, a Beilstein Institute computerized system for the generation of IUPAC systematic nomenclature.

The phrase "as defined hereinabove" refers to the first definition provided in the Detailed Description of the Invention.

The term "alkyl" as used herein denotes an unbranched or branched chain, saturated, monovalent hydrocarbon residue containing 1 to 10 carbon atoms. "C₁₋₁₀ alkyl" as used herein refers to an alkyl composed of 1 to 10 carbons. Examples of alkyl groups include, but are not limited to, lower alkyl groups include methyl, ethyl, propyl, *i-*propyl, n-butyl, *i*-butyl, *t*-butyl or pentyl, isopentyl, neopentyl, hexyl, heptyl, and octyl.

The term "alkoxy group" as used herein means an -O-alkyl group, wherein alkyl is as defined above such as methoxy, ethoxy, *n*-propyloxy, *i*-propyloxy, *n*-butyloxy, *i-*butyloxy, *t*-butyloxy, pentyloxy, hexyloxy, including their isomers. "C₁₋₁₀ alkoxy" as used herein refers to an-O-alkyl wherein alkyl is C₁₋₁₀-alkyl.

The term "acyl" as used herein denotes a group of formula -C(=O)R wherein R is hydrogen, alkyl as defined herein or phenyl. The term or "alkylcarbonyl" as used herein denotes a group of formula C(=O)R wherein R is alkyl as defined herein. The term "arylcarbonyl" as used herein means a group of formula C(=O)R wherein R is an aryl group; the term "benzoyl" as used herein an "arylcarbonyl" group wherein R is phenyl.

The term "acylating agent" as used herein refers a compound capable of introducing an acyl group as defined above into a molecule. Acylating agents which react with hydroxyl, amine or sulfur substiuents commonly are either carboxylic acid anhydrides or acyl halides. The term "anhydride" as used herein refers to compounds of the general structure RC(O)-O-C(O)R wherein is as defined in the previous paragraph. The term "acyl halide" as used herein refers to the group RC(O)X wherein X is bromo or chloro. One skilled in the art will recognize that other "activated derivatives" of carboxylic acids are known in the art and these can also be used. The term "activated derivative" of a compound as used herein refers to a transient reactive form of the original compound which renders the compound reactive in a desired chemical reaction, in which the original compound is only moderately reactive or non-reactive.

The term "arylalkyl" or "aralkyl" as used herein denotes the radical R'R"-, wherein R' is an aryl radical and R" is an alkylene radical with the understanding that the attachment point of the arylalkyl moiety will be on the alkylene radical. The term "alkylene" as used herein denotes a divalent linear or branched saturated hydrocarbon radical, having from one to six carbons inclusive. The term aryl as used herein refers to a phenyl, a 1-naphthyl or a 2-napthyl moiety. Examples of alkylene radicals include, but are not limited to, methylene, ethylene, propylene, 2-methyl-propylene, butylene, 2-ethylbutylene. Examples of arylalkyl radicals include, but are not limited to, benzyl, phenylethyl and 3-phenylpropyl.

The term "haloalkyl" as used herein denotes a unbranched or branched chain alkyl group as defined above wherein 1, 2, 3 or more hydrogen atoms are substituted by a halogen. Examples are 1-fluoromethyl, 1-chloromethyl, 1-bromomethyl, 1-iodomethyl, trifluoromethyl, trichloromethyl, tribromomethyl, triiodomethyl, 1-fluoroethyl, 1-chloroethyl, 1-bromoethyl, 1-iodoethyl, 2-fluoroethyl, 2-chloroethyl, 2-bromoethyl, 2-iodoethyl, 2,2-dichloroethyl, 3-bromopropyl or 2,2,2-trifluoroethyl.

The term "halogen" or "halo" as used herein refers to fluorine, chlorine, bromine, or iodine.

The term "phase transfer catalyst" as used herein refers to a catalyst which alters the rate of transfer of water-soluble reactant across the interface to the organic phase where the anion or neutral compound can freely react with the organic reactant already located in the organic phase. Phase transfer catalysts commonly employed are quaternary ammonium salts, phosphonium salts and crown ethers or polyethylene glycols. Suitable catalysts are, e.g., tetrabutylammonium chloride, tetrabutylammonium bromide, tetrabutylphosphonium chloride, benzyl triethylammonium chloride, and N-2-ethylhexyl-4-dimethylamino pyridinium bromide. The term "quaternary ammonium azide" refers to a species R¹R²R³R⁴N⁺ N₃⁻ wherein R¹ to R⁴ are independently alkyl or aralkyl.

The phrase "polar aprotic solvent" as used herein denotes polar solvents which sufficiently polar to dissolve nucleoside derivatives but lack an exchangeable proton such as acetonitrile, DMF, dioxane, tetrahydrofuran, and the like.

The phrase "nonpolar organic solvent" as used herein means organic solvents such as, ligroin, pentane, hexane, cyclohexane, heptane, octane, benzene, toluene, diethyl ether, dioxane, tetrahydrofuran, dichloromethane, carbon tetrachloride, and the like.

The term "halogenating agent" as used herein refers to a reagent capable of converting an alcohol to an alkyl halide. The term "dehydrohalogenating agent" as used here refers to a compound capable of effecting the elimination of a hydrohalic acid HX from a haloalkane to afford an alkene.

The term "iodo azide" as used herein refers to two adjacent carbon atoms substituted by an iodide and by an azide, i.e. I-CH₂C(N₃)=.

The term "first base" refers to a base capable of reacting with an ester to afford the corresponding alcohol and carboxylic acid. Suitable first bases include ammonia, primary and secondary amines, NaHCO₃, Na₂CO₃, KOH and NaOMe and the like. The term "second base" refers to a base used in an acylation reaction as a catalyst and reagent to remove acid liberated during the transformation. Typical second bases include TEA, pyridine, BMAP, NMM, DABCO and the like.

The term "nucleoside" as used herein refers to a nitrogenous heterocyclic base linked to a pentose sugar by a glycosidic bond at C-1. Naturally occurring bases include uracil, thymine, cytosine, adenine and guanine and naturally occurring sugars are ribose and 2-deoxyribose. The term nucleoside further encompasses compounds in which the sugar and/or the nitrogenous base have been chemically modified.

As used herein, the term "treating", "contacting" or "reacting" when referring to a chemical reaction means to add or mix two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

### ABBREVIATIONS

- BTEAA: benzyl triethylammonium azide
- DABCO: 1,4-diazabicyclo [2.2.2] octane
- DBN: 1,8-diazabicyclo[4.3.0]non-5-ene
- DCM: dichloromethane
- DMF: N,N-dimethylformamide
- DMAP: 4-dimethylaminopyridine
- Gc: gas chromatography
- HMPA: hexamethylphosphoramide
- Hplc: high performance liquid chromatography
- MCPBA: *m*-chloroperbenzoic acid
- MCBA: *m*-chlorobenzoic acid
- MeCN: acetonitrile
- NMM: N-methylmorpholine
- Ph: phenyl
- TEA: triethylamine
- THF: tetrahydrofuran
- TPP: triphenylphosphine

Although specific methods for producing 4'-azidonucleoside derivatives are described below, numerous modifications and alternative process steps will be apparent to those skilled in the art. Accordingly, this description and these examples are to be construed as illustrative only and is for teaching those skilled in the art novel processes for producing 4'-azidonudeoside derivatives. These processes may be varied substantially without departing from the spirit of the invention and the exclusive use of all modifications which come within the scope of the appended claim is reserved.

### EXAMPLE 1

### 1-((2R,3R,4S,5S)-3,4-Dihydroxy-5-iodomethyl-tetrahydro-furan-2-yl)-1H-pyrimidine-2,4-dione (IVh)

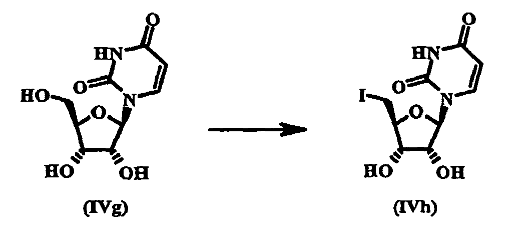

Uridine (IVg, 30.0 kg), TPP (46.8 kg) and imidazole (12.2 kg) were slurried in THF (267 kg). A solution of iodine (33.2 kg) in THF (87 kg) was added slowly to the slurry while the reaction temperature was maintained below 28° C. The reaction mixture was stirred overnight (*ca.* 18 h) at about 25° C to achieve complete conversion. The reaction mixture was quenched with a small amount (2.3 L) of water. The reaction mixture was distilled under moderate vacuum while adding isopropanol (maximum internal temperature: 50° C) till IPA content (by gc) of the distillate was greater than 87% (v/v). The resulting slurry was cooled to room temperature (*ca.* 22° C) and aged overnight. The precipitated product was filtered and washed with isopropanol (2 x 50 kg) and dried at about 50° C under vacuum with a slow nitrogen stream to afford IVh (36.5 kg; 83.9% theory.).

### EXAMPLE 2

### 1-((2R,3R,4S)-3,4-Dihydroxy-5-methylene-tetrahydro-furan-2-yl)-1H-pyrimidine-2,4-dione (Vc)

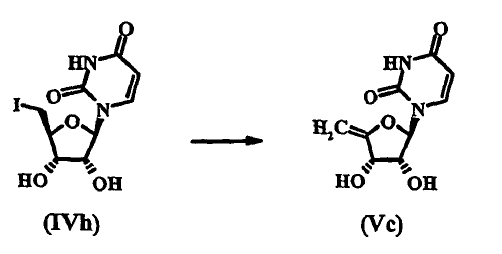

A suspension of IVh (4.0 kg) in MeOH (20 kg) was treated with 25% sodium methoxide solution (6.1 kg) to obtain a clear solution, which was maintained at *ca.* 60° C for about 2 h. The methanol was removed via vacuum distillation and replaced with MeCN until the methanol content (by gc) dropped to about 0.5% v/v. The volume of the resulting slurry was adjusted to about 30 L with acetonitrile and then treated with 3.5 kg of acetic anhydride and heated at about 60° C for *ca.* 5 h. The reaction mixture was concentrated *in vacuo* to about 18 L and diluted with ethyl acetate. The reaction mixture was cooled to *ca.* 20° C and excess Ac₂O quenched by slow addition of a saturated NaHCO₃ until the pH of the solution was *ca.* 7.5. The phases were separated and the organic phase was washed with water and brine. The organic phase was concentrated *in vacuo,* diluted with MeOH (19 kg ) and treated with 1.08 kg of con. NH₄OH. The reaction mixture was allowed to stand overnight (*ca.* 18 h) at about 20° C to complete the deacetylation. The reaction mixture was concentrated *in vacuo* (internal temp <40° C) and the residue diluted with a mixture of isopropanol and acetonitrile. The final composition of solvent at the end of displacement distillation was typically: acetonitrile 65%, IPA 30%, methanol 5%. The olefin Vc precipitated and the resulting slurry was cooled to *ca.* 15° C, filtered, washed with cold acetonitrile and dried under *in vacuo* at 20-25° C to yield Vc (1.92 kg; 75.1% theory).

### EXAMPLE 3

### Benzoic acid (2S,3S,4R,5R)-4-benzoyloxyy-2-azido-5-(2,4-dioxo-3,4-dihydro-2H-pyrimidin-1-yl)-2-iodomethyl-tetrahydro-furan-3-yl ester (IIg)

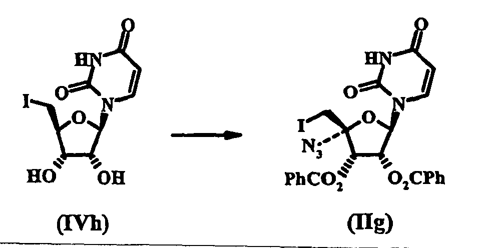

A suspension of IVh (12.0 kg) in MeOH (68 kg) was treated with 25% sodium methoxide solution (18.4 kg) to obtain a clear solution, which was allowed to stand at about 60°C for about 2 h to achieve complete conversion. The reaction mixture was then added to a solution of N-methylinorpholinium mesylate in methanol (prepared in situ by adding 8.9 kg of NMM to a solution of 8.1 kg of methanesulfonic acid in 19 kg of MeOH). The reaction mixture was concentrated *in vacuo* (internal temp <40°C) and the evaporated MeOH was replaced with THF (batch volume ca. 50 L) to until the residual methanol level was *ca.* 1-2% (by gc). The resulting slurry of crude Vc was diluted with acetonitrile (20 kg) and made slightly basic with NMM (1.2kg). Benzyl triethylammonium chloride (10.0 kg) and sodium azide (2.87 kg) were slurried together in acetonitrile (45 kg) to extract azide into acetonitrile as the quaternary ammonium azide. The slurry was filtered, and the quaternary azide solution was added to the slurry of crude Vc. A solution of iodine (11.2 kg) in THF (40 kg) was then added slowly to the resulting slurry while maintaining batch temperature at 0-5°C. After completion of addition, the reaction mixture was allowed to stand at 5-10° C for 18-24 hours to complete the conversion. To the reaction mixture was added TEA (17.2 kg) and DMAP (0.41 kg), and the mixture cooled to about -10°C and treated with benzoyl chloride (14.3 kg) while maintaining the internal temperature below -5°C. After the addition was completed, the reaction mixture was allowed to stand at *ca*. -5°C until benzoylation was complete. The reaction mixture was quenched with water and aqueous sodium sulfite (to destroy residual iodine) solution and treated with EtOAc (44 kg) was added. The organic phase was washed with water and water back-extracted with EtOAc (44 kg) and the combined organic extracts concentrated under reduced pressure (maximum jacket temperature: 65°C) and the evaporated solvents were replaced with isopropanol from which IIg crystallized. The resulting slurry is cooled to *ca.* 20° C and allowed to stand for at least 2 h. The precipitated product was isolated by filtration, washed with isopropanol and dried at 25-50° C under a vacuum in a stream of nitrogen to yield IIg (15.9 kg; overall yield 77.6% theoretical)

### EXAMPLE 4

### Benzoic acid (2S,3S,4R,5R)-4-benzoyloxyy-2-azido-5-(2,4-dioxo-3,4-dihydro-2H-pyrimidin-1-yl)-2-iodomethyl-tetrahydro-furan-3-yl ester (IIg)

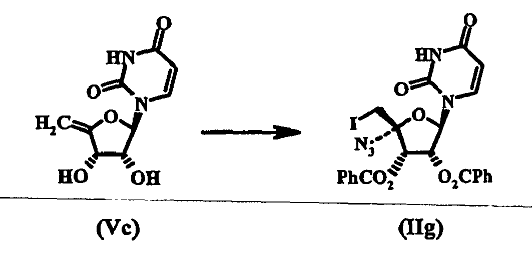

### step 1

A mixture of benzyl triethylammonium chloride (2.0 kg) and sodium azide (0.69 kg) was slurried together in MeCN (12 kg). The insoluble sodium chloride was removed by filtration and the filtrate washed with MeCN. To a homogenous mixture of Vc (1.9 kg), 4-NMM (0.26 kg) and THF (7.6 L) was added the MeCN solution of benzyl triethylammonium azide which produced a clear solution. A solution of iodine (2.45 kg) in THF (10 L) was added slowly while maintaining the internal temperature at 0-5° C. After the addition was completed the reaction mixture was aged at 5-10° C for *ca.* 2 h. Excess azide was destroyed by adding small amounts of N-acetyl cysteine (40 g) before proceeding.

### step 2

The crude reaction mixture from the previous step was treated with NMM (4.3 kg) and DMAP (100 g), cooled to about 0°C and benzoyl chloride (2.6 kg) was added while maintaining internal temperature at *ca.* 5° C. After the addition was complete, the reaction mixture was stirred at *ca.* 5° C for *ca.* 30 min. Water was carefully added to destroy excess benzoyl chloride, sodium sulfite was added to destroy residual iodine and EtOAc was added to extract desired product. The EtOAc solution was washed with water and concentrated *in vacuo.* The EtOAc thus removed was replaced by isopropanol (maximum jacket temperature: 65°C) which resulted in crystallization of the desired product. The resulting slurry was cooled to ca. 22° C and allowed to stand overnight. The precipitate filtered, washed with isopropanol and dried at room temperature *in vacuo* under a stream of nitrogen to yield IIg (3.80 kg; 74.2% theory)

### EXAMPLE 5

### 3-Chloro-benzoic acid (2R,3S,4R,5R)-2-azido-3,4-bis-benzoyloxy-5-(2,4-dioxo-3,4-dihydro-2H-pyrimidin-1-yl)-tetrahydro-furan-2-ylmethyl ester (ITId)

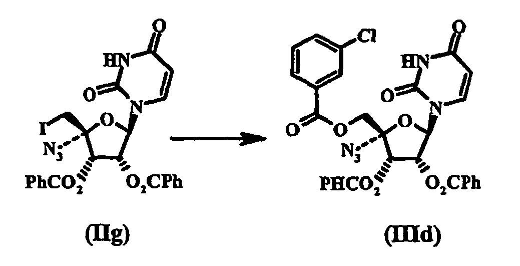

A mixture of IIg (14.2 kg), tetrabutyl ammonium hydrogen sulfate (8.5 kg), potassium hydrogen phosphate (8.5 kg), *m*-chlorobenzoic acid (4.0 kg), DCM (70 kg) and water (28 kg) was charged to a slurry of m-chloroperbenzoic acid (22.4 kg) in DCM (70 kg). The mixture was stirred at room temperature until the reaction was complete (by HPLC). To quench the reaction, the reaction mixture a solution of sodium sulfite (19 kg) in water (70 kg) was added while maintaining temperature below 25° C. After a stirring for a short time, a solution of potassium carbonate (28 kg) in water (51 kg) was added. The lower organic layer is separated and concentrated under atmospheric pressure. The DCM was replaced with isopropanol. The resulting solution (vol. 40 -50 L) was treated with hot water (70 L) which resulted in the precipitation of the desired product. The resulting slurry was warmed to about 65° C for 2 h and then allowed to cool to room temperature. The precipitated product was isolated by filtration, washed with a mixture of isopropanol and water and dried under vacuum at about 50°C to afford IIId (10.6 kg; 71.3% theory)

### EXAMPLE 6

### 3-Chloro-benzoic acid (2R,3S,4R,5R) -2-azido-3,4-bis-benzoyloxy-5-(2-oxo-4-[1,2,4]triazol-1-yl-3,4-dihydro-2H-pydmidin-1-yl)-tetrahydro-furan-2-ylmethyl ester (IIIe)

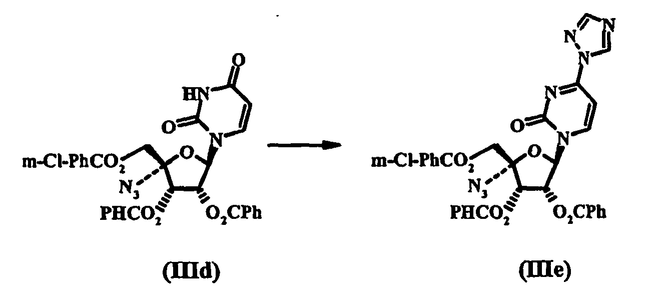

Phosphorous oxychloride (21.5 kg) was added to a cooled mixture of IIId (34.1 kg), 1,2,4-triazole (42.1 kg), TEA (63.1 kg) and DCM (270 kg) while the reaction temperature was maintained below 25° C. The reaction mixture was stirred at rt and the progress of the reaction monitored by hplc. When the reaction was complete the excess POCl₃ was quenched by careful addition of cold water (280 kg) while the reaction temperature was maintained below 30°C. The lower organic layer is separated and concentrated by distillation under atmospheric pressure. As the DCM distilled it was replaced by MeCN to maintain the volume approximately constant. The resulting slurry was (*ca.* 70 L) was diluted with water (70 L) which resulted in the precipitation of IIIe The resulting slurry was stirred at approximately 15° C for up to 16 h. The precipitate was isolated by filtration, washed with a mixture of acetonitrile and water and dried under vacuum at about 50°C to yield IIIe (32.7 kg; 88.8% theory).

### EXAMPLE 7

### 1-((2R,3R,4S,5R)-5-Azido-3,4-dihydroxy-5-hydroxymethyl-tetrahydro-furan-2-yl)-2-oxo-1,2-dihydro-pyrimidin-4-yl-ammonium; hydrogen sulfate (VIIIc)

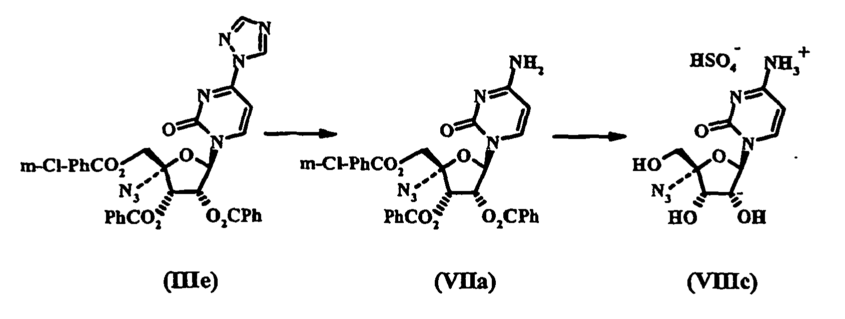

Triazole IIIe (32.2 kg) was suspended in THF (152 kg) and treated with conc. aqueous ammonium hydroxide (15 kg). After the ammonolysis reaction was completed, the reaction mixture was concentrated *in vacuo* and the methanol was added to bring the volume to *ca.* 180 L. To the resulting solution was added conc. aqueous ammonium hydroxide (15 kg) to cleave the protective ester functionality. After completion of the desired reaction, the solution was filtered, concentrated *in vacuo* and the reaction mixture was diluted with isopropanol to *ca.* 80 L. The resulting solution was diluted with isopropanol (64 kg) and water (for irrigation) (82 kg) which produced a clear solution. While warming up the solution to about 70° C, the solution was treated with dilute aqueous sulfuric acid -isopropanol mixture (prepared by mixing 2.4 kg of conc. sulfuric acid with 10 kg of water followed by addition of 68 kg of isopropanol). The resulting slurry is aged at ca. 70° C for about 0.5 h and then cooled to ambient temperature over 2 hours. The precipitated product was isolated by filtration, washed with isopropanol and dried *in vacuo* with a nitrogen stream at about 50° C to yield VIIIa (15.3 kg; 95.8% theory)

The steps described in claim 8 represent the best mode of carrying out the process known to the inventors. These are described in Examples 1,3,5-7 respectively. These steps provide the most convenient and economical mode to practice the invention.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

All patents, patent applications and publications cited in this application are hereby incorporated by reference in their entirety for all purposes to the same extent as if each individual patent, patent application or publication were so individually denoted.

## Claims

1. Process for the preparation of a 4'-azido-cytidine compound of formula VIII 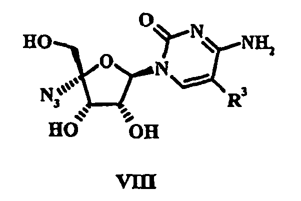 and pharmaceutically acceptable addition salts thereof
wherein
R³ is H, C₁₋₆-alkyl, C₁₋₃-haloalkyl or halogen
**characterized in that**
in step a)
nucleoside compound of formula VIa 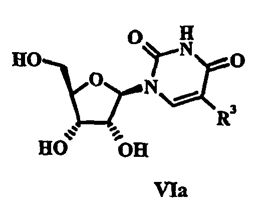 is transformed to a compound of formula II 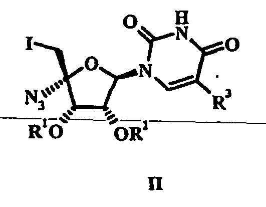 wherein
R¹ is R^{1a}CO-;
R^{1a} is C₁₋₁₀-alkyl or phenyl optionally substituted with 1 to 3 substituents selected from the group consisting of alkyl, alkoxy, halogen, nitro or cyano;
R³ is as defined above,
in step b)
a solution of 5'-iodo compound of formula II in a suitable solvent is contacted with a peracid, R^{2a}C(O)OOH, an acid R^{2a}C(O)OH and optionally a phase transfer catalyst to produce a 4'-azido-2',3',5'-triaryl-nucleoside compound of formula I 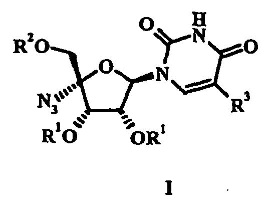 wherein
R¹ is R^{1a}CO-;
R² is R^{2a}CO-;
R^{1a} and R^{2a} are independently C₁₋₁₀-alkyl or phenyl optionally substituted with 1 to 3 substituents selected from the group consisting of alkyl, alkoxy, halogen, nitro or cyano
R³ is as defined above,
in step c)
the 4'-azido-2',3',5'-triacyl-nucleoside compound of formula I is transformed to afford a 4'-azido-cytidine compound of formula VIII and if necessary, a pharmaceutically acceptable addition salts.

2. Process for preparation of a 4'-azido-2',3',5'-triaryl-nucleoside of formula I, comprising contacting a solution of 5'-iodo compound of formula II in a suitable solvent is contacted with a peracid, R^{2a}C(O)OOH, an acid R^{2a}C(O)OH and optionally a phase transfer catalyst to produce a 4'-azido-2',3',5'-triacyl-nudeoside compound of formula I 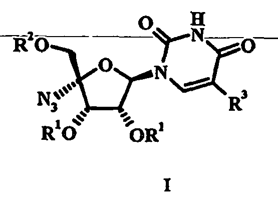 wherein
R¹ is R^{1a}CO-;
R² is R^{2a}CO-;
R^{1a} and R^{2a} are independently C₁₋₁₀-alkyl or phenyl optionally substituted with 1 to 3 substituents selected from the group consisting of alkyl, alkoxy, halogen, nitro or cyano;
R³ is H, C₁₋₆-alkyl. C₁₋₃-haloalkyl or halogen.

3. Process according to claim 1 for the preparation of the 4'-azido-2',3',5'-triacyl-nudeoside compound of formula Ia, **characterized in that** said process further comprises contacting said 4'-azido-2',3',5'-triacyl-nucleoside compound of formula IV with a first base to afford a 4'-azido-nucleoside compound of formula Ia 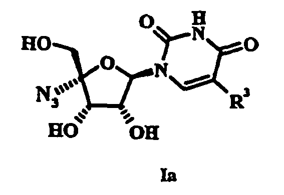 wherein R³ is as defined above.

4. Process according to claim 1 for preparing a 5'-iodo compound of formula II, **characterized in that** said process comprises the steps of
a) contacting nucleoside IVa with an halogenating agent to produce a 5'-halo nucleoside compound of formula IVb 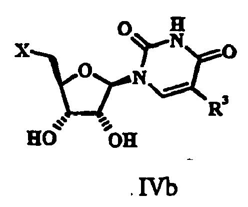 wherein X is a halogen, R³ is as defined in claim 1;
b) contacting compound of formula IVb with a dehydrohalogenating agent to produce a 5-methylene nucleoside compound Va 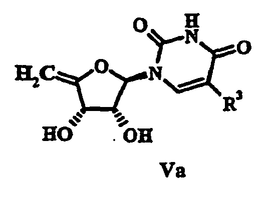 wherein R³ is as defined above;
c) contacting compound of formula Va with a quaternary ammonium azide and iodine dissolved in tetrahydrofuran(THF) and acetonitrile(MeCN) to produce an iodo azide compound of formula IIa 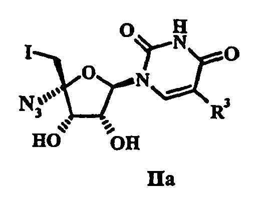 wherein R³ is as defined above;
d) contacting compound of formula IIa with at least one second base and a first acylating agent to afford diester compound of formula II 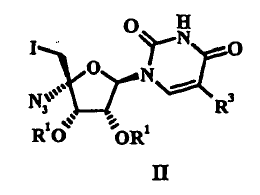
wherein R¹ and R³ are as defined in claim1.

5. Process for preparing 4'-azidocytidine compound of formula VIII according to claim 1, **characterized in that** said process comprises the steps of:
a) contacting compound of formula I 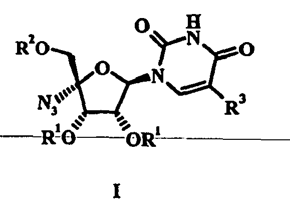 wherein R1 and R2 are defined in claim 1, R³ is hydrogen with 1,2,4-triazole, phosphorus oxychloride, triethylamine(TEA) in CH₂Cl₂ to afford triazole compound of formula VI 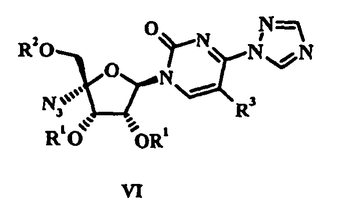 wherein R¹, R² and R³ are as defined above;
b) contacting compound of formula VI with a solution of ammonium hydroxide and tetrahydrofuran(THF) to displace the triazole to afford a 4'-azido-2',3',5'-triacylcytidine compound of formula VII 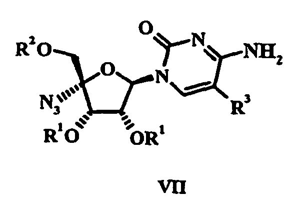 wherein R¹, R² and R³ are as defined above;
c) contacting compound of formula VII with a solution of ammonia and an alcohol to cleave the esters to afford compound of formula VIII 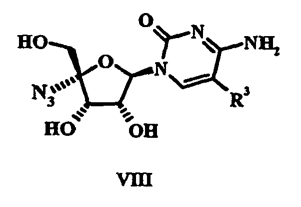
wherein R¹, R² and R³ are as defined above.

6. Process according to claim 4, **characterized in that** said process comprises the steps of:
a) contacting compound of formula Va 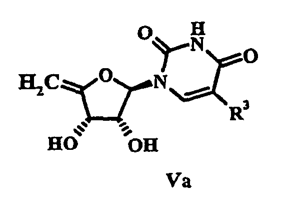 with a second acylating agent and optionally with a trialkylamine base to afford diacyl compound Vb 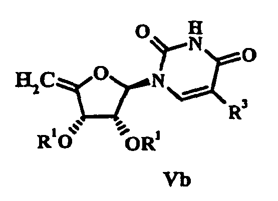 wherein R¹ and R³ are as defined in claim 4,
and washing an organic solution of Vb with aqueous NaHCO₃ wherein the pH was maintained at about 7.5; and,
b) contacting said diacyl compound of formula Vb with a solution of ammonia and methanol to regenerate Va.

7. Process according to claim 1 for preparing the hemisulfate acid addition salt of 4'-azidocytidine of formula VIIIa 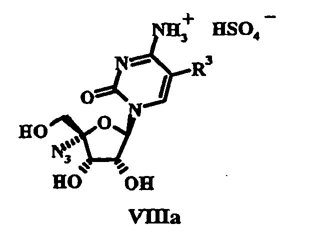 **characterized in that** said process further comprises the crystallizing a compound of formula VIII from isopropanol, water and sulfuric acid.

8. Process according to claim 7 for the preparation of the hemisulfate acid addition salt of 4'-azido-cytidine of formula VIIIa wherein R³ is hydrogen; R¹ is PhCO; R² is R^{2a}CO wherein R^{2a} is optionally substituted phenyl; said phase transfer catalyst is a tetraalkyl ammonium hydrogen sulfate, said solvent is mixture of an aqueous buffer and a nonpolar organic solvent, said iodinating agent is triphenylphosphine(TPP), iodine and imidazole; said dehydrohalogenating agent is sodium methoxide or 1,8-diazabicyclo[4.3.0]non-5-ene(DBN); said quaternary ammonium azide is benzyl triethylammonium azide and said acylating agent is benzoyl chloride, said first base is ammonia and said second base is a N-methylmorpholine(NMM)and 4-dimethylaminopyridine(DMAP) and said alcohol is methanol.

9. Process for the preparation of 4'-azidocytidine hemisulfate of formula VIIIc 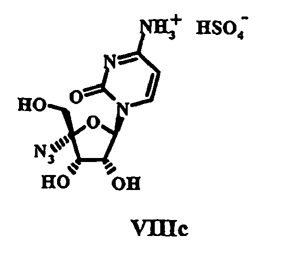 **characterized in that** said process comprises the steps of:
a) contacting a tetrahydrofuran(THF) solution of uridine compound of formula IVg 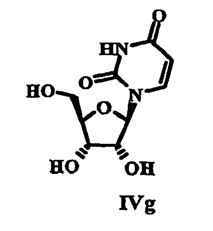 with triphenylphosphine(TPP), iodine and imidazole to produce 1-(3,4-dihydroxy-5-halomethyl-tetrahydro-furan-2-yl)-1H-pyrinudme-2,4-dione of formula IVh 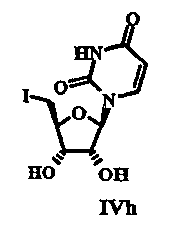
b) contacting compound of formula IVh with a methanolic solution of sodium methoxide agent to produce 1-(3,4-dihydroxy-5-methylene-tetrahydro-furan-2-yl)-1H-pyrimidine-2,4-dione of formula Vc; 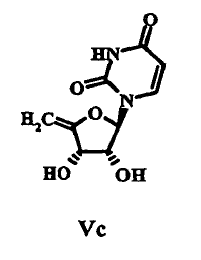
c) contacting compound of formula Vc with a solution of benzyl triethylammonium azide and iodine in tetrahydrofuran(THF) and acetonitrile(MeCN) to afford iodo azide of formula IIe; 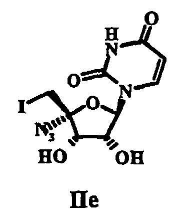
d) contacting compound of formula IIe with a solution benzoyl chloride, N-methylmorpholine(NMM) and acetonitrile(MeCN) to afford the dibenzoate compound of formula IIg: 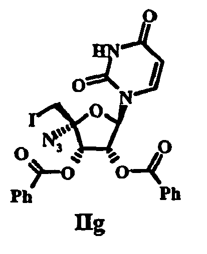
e) contacting compound of formula IIg with *m*-chloroperbenzoic acid, *m*-chlorobenzoic acid and tetrabutylammonium hydrogen sulfate in a two-phase solution of dichloromethane(DCM) and an aqueous solution of potassium hydrogen phosphate to afford compound of formula IIId; 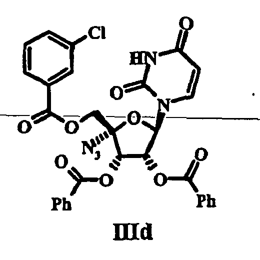
f) contacting compound of formula IIId with a solution of 1,2,4-triazole, phosphorus oxychloride, triethylamine(TEA) in dichloromethane(DCM) to afford compound of formula IIIe; 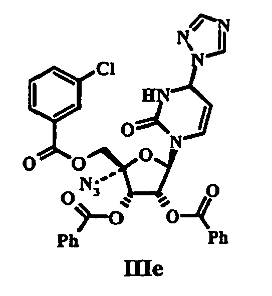
g) contacting compound of formula IIIe with methanol solution of ammonia to cleave the esters and afford free base of compound of formula VIIIb; 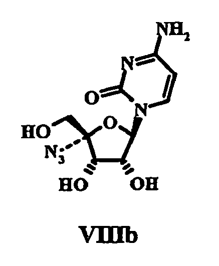
h) crystallize compound of formula VIIIb from isopropanol and water containing H₂SO₄ to afford the hemisulfate salt of formula VIIIc.

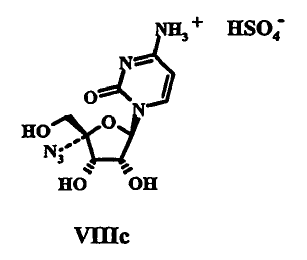

10. A compound according to formula Ic 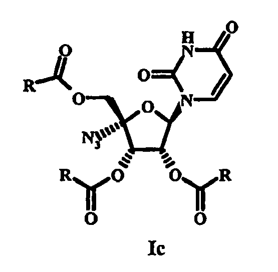 wherein R is phenyl optionally substituted with one to three substituents selected from the group consisting of halogen, C₁₋₁₀-alkyl, C₁₋₁₀-alkoxy, nitro, cyano.

## Patentansprüche

1. Verfahren zur Herstellung einer 4'-Azido-cytidin-Verbindung der Formel VIII 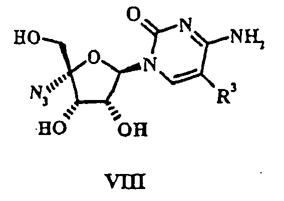 und pharmazeutisch akzeptablen Additionssalzen davon,
worin
R³ H, C₁₋₆-Alkyl, C₁₋₃-Halogenalkyl oder Halogen ist
**dadurch gekennzeichnet, daß**
in Schritt a)
die Nucleosidverbindung der Formel VIa 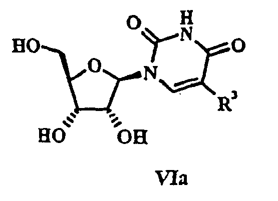 zu einer Verbindung der Formel II 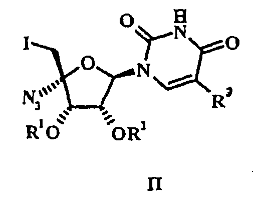 umgewandelt wird, worin
R¹ R^{1a}CO- ist;
R^{1a} C₁₋₁₀-Alkyl oder Phenyl ist, gegebenenfalls substituiert mit 1 bis 3 Substituenten, ausgewählt aus der Gruppe, bestehend aus Alkyl, Alkoxy, Halogen, Nitro oder Cyano;
R³ wie oben definiert ist,
in Schritt b)
eine Lösung der 5'-Iodverbindung der Formel II in einem geeigneten Lösungsmittel mit einer Persäure, R^{2a}C(O)OOH, einer Säure R^{2a}C(O)OH und gegebenenfalls einem Phasentransferkatalysator unter Herstellung einer 4'-Azido-2',3',5'-triacyl-nucleosid-Verbindung der Formel I kontaktiert wird, 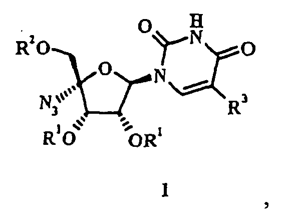 worin
R¹ R^{1a}CO- ist;
R² R^{2a}CO- ist;
R^{1a} und R^{2a} unabhängig C₁₋₁₀-Alkyl oder Phenyl sind, gegebenenfalls substituiert mit 1 bis 3 Substituenten, ausgewählt aus der Gruppe, bestehend aus Alkyl, Alkoxy, Halogen, Nitro oder Cyano;
R³ wie oben definiert ist,
in Schritt c)
die 4'-Azido-2',3',5'-triacyl-nucleosid-Verbindung der Formel I umgewandelt wird, um eine 4'-Azido-cytidin Verbindung der Formel VIII und gegebenenfalls pharmazeutisch akzeptable Additionssalze zu erhalten.

2. Verfahren zur Herstellung eines 4'-Azido-2',3',5'-triacyl-nucleosids der Formel I, umfassend das Kontaktieren einer Lösung der 5'-Iodverbindung der Formel II in einem geeigneten Lösungsmittel mit einer Persäure, R^{2a}C(O)OOH, einer Säure R^{2a}C(O)OH und gegebenenfalls einem Phasentransferkatalysator unter Herstellung einer 4'-Azido-2',3',5'-triacyl-nucleosid-Verbindung der Formel I 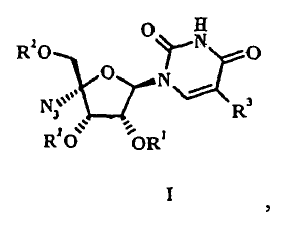 worin
R¹ R^{1a}CO- ist;
R² R^{2a}CO- ist,
R^{1a} und R^{2a} unabhängig C₁₋₁₀-Alkyl oder Phenyl sind, gegebenenfalls substituiert mit 1 bis 3 Substituenten, ausgewählt aus der Gruppe, bestehend aus Alkyl, Alkoxy, Halogen, Nitro oder Cyano;
R³ H, C₁₋₆-Alkyl, C₁₋₃-Halogenalkyl oder Halogen ist.

3. Verfahren nach Anspruch 1 zur Herstellung der 4'-Azido-2',3',5'-triacyl-nucleosid-Verbindung der Formel Ia, **dadurch gekennzeichnet, daß** das Verfahren außerdem das Kontaktieren der 4'-Azido-2',3',5'-triacyl-nucleosid-Verbindung der Formel IV mit einer ersten Base umfaßt, um eine 4'-Azido-nucleosid-Verbindung der Formel Ia zu erhalten, 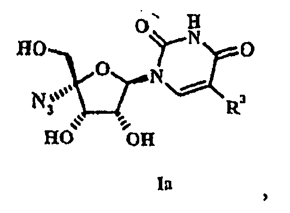 worin R³ wie oben definiert ist.

4. Verfahren nach Anspruch 1 zur Herstellung einer 5'-Iodverbindung der Formel II, **dadurch gekennzeichnet, daß** das Verfahren die Schritte umfaßt:
a) Kontaktieren des Nucleosids IVa mit einem Halogenierungsmittel unter Herstellung einer 5'-Halogen-nucleosid-Verbindung der Formel IVb 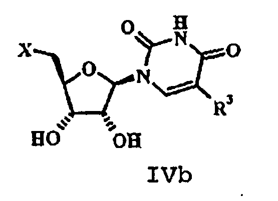 worin X ein Halogen ist, R³ wie in Anspruch 1 definiert ist;
b) Kontaktieren der Verbindung der Formel IVb mit einem Dehydrohalogenierungsmittel unter Herstellung einer 5-Methylennucleosid-Verbindung Va 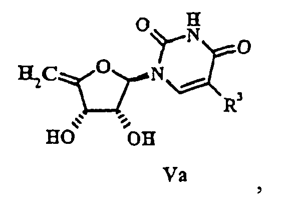 worin R³ wie oben definiert ist;
c) Kontaktieren der Verbindung der Formel Va mit einem quartären Ammoniumazid und Iod, gelöst in Tetrahydrofuran (THF) und Acetonitril (MeCN), unter Herstellung einer Iodazidverbindung der Formel IIa 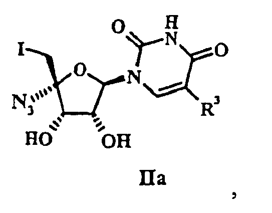 worin R³ wie oben definiert ist;
d) Kontaktieren der Verbindung der Formel IIa mit mindestens einer zweiten Base und einem ersten Acylierungsmittel, um eine Diesterverbindung der Formel II zu erhalten, 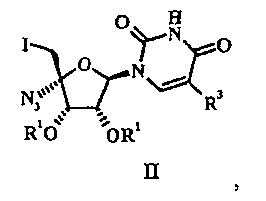
worin R¹ und R³ wie in Anspruch 1 definiert sind.

5. Verfahren zur Herstellung einer 4'-Azidocytidinverbindung der Formel VIII nach Anspruch 1, **dadurch gekennzeichnet, daß** das Verfahren die Schritte umfaßt:
a) Kontaktieren der Verbindung der Formel I 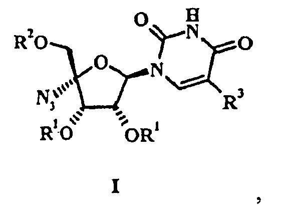 worin R¹ und R² wie in Anspruch 1 definiert sind, R³ Wasserstoff ist, mit 1,2,4-Triazol, Phosphor(V)-oxidchlorid, Triethylamin (TEA) in CH₂Cl₂, um eine Triazolverbindung der Formel VI zu erhalten, 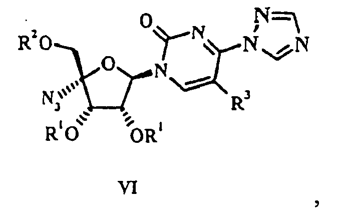 worin R¹, R² und R³ wie oben definiert sind;
b) Kontaktieren der Verbindung der Formel VI mit einer Lösung aus Ammoniumhydroxid und Tetrahydrofuran (THF), um das Triazol zu verdrängen, um eine 4'-Azido-2',3',5'-triacyl-cytidinverbindung der Formel VII zu erhalten, 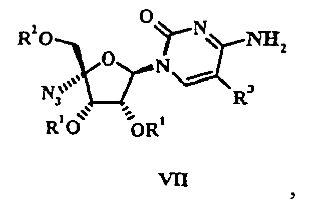 worin R¹, R² und R³ wie oben definiert sind;
c) Kontaktieren der Verbindung der Formel VII mit einer Lösung aus Ammoniak und einem Alkohol, um die Ester zu spalten, um eine Verbindung der Formel VIII zu erhalten, 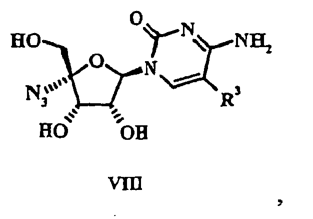
worin R¹, R² und R³ wie oben definiert sind.

6. Verfahren nach Anspruch 4, **dadurch gekennzeichnet, daß** das Verfahren die Schritte umfaßt:
a) Kontaktieren der Verbindung der Formel Va 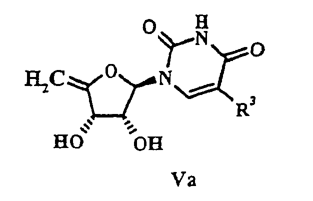 mit einem zweiten Acylierungsmittel und gegebenenfalls mit einer Trialkylaminbase, um eine Diacylverbindung Vb zu erhalten, 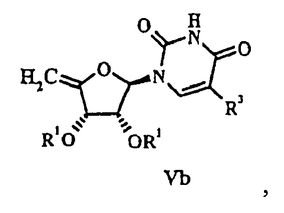 worin R¹ und R³ wie in Anspruch 4 definiert sind,
und das Waschen einer organischen Lösung von Vb mit wässerigem NaHCO₃, wobei der pH bei etwa 7,5 gehalten wird; und
b) Kontaktieren der Diacylverbindung der Formel Vb mit einer Lösung aus Ammoniak und Methanol unter Regenerierung von Va.

7. Verfahren nach Anspruch 1 zur Herstellung des Hemisulfatsäureadditionssalzes des 4'-Azidocytidins der Formel VIIIa, 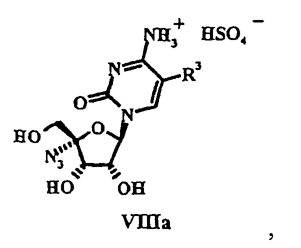 **dadurch gekennzeichnet, daß** das Verfahren, außerdem die Kristallisierung einer Verbindung der Formel VIII aus Isopropanol, Wasser und Schwefelsäure umfaßt.

8. Verfahren nach Anspruch 7 zur Herstellung des Hemisulfatsäureadditionssalzes des 4'-Azido-cytidins der Formel VIIIa, worin R³ Wasserstoff ist; R¹ PhCO ist; R² R^{2a}CO ist,
worin R^{2a} gegebenenfalls substituiertes Phenyl ist; der Phasentransferkatalysator ein Tetraalkylammoniumhydrogensulfat ist, das Lösungsmittel ein Gemisch aus einem wässerigen Puffer und einem nicht-polaren organischen Lösungsmittel ist, das Iodierungsmittel Triphenylphosphin (TPP), Iod und Imidazol ist; das Dehydrohalogenierungsmittel Natriummethoxid oder 1,8-Diazabicyclo[4.3.0]non-5-en (DBN) ist; das quartäre Ammoniumazid Benzyltriethylammoniumazid ist und das Acylierungsmittel Benzoylchlorid ist, die erste Base Ammoniak ist und die zweite Base ein N-Methylmorpholin (NMM) und 4-Dimethylaminopyridin (DMAP) ist und der Alkohol Methanol ist.

9. Verfahren zur Herstellung des 4'-Azidocytidinhemisulfats der Formel VIIIc 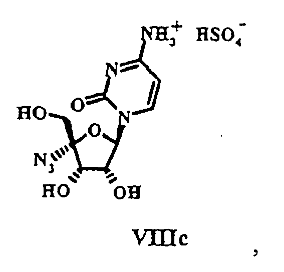 **dadurch gekennzeichnet, daß** das Verfahren die Schritte umfaßt:
a) Kontaktieren einer Tetrahydrofuranlösung (THF-Lösung) einer Uridinverbindung der Formel IVg 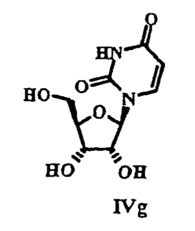 mit Triphenylphosphin (TPP), Iod und Imidazol unter Herstellung von 1-(3,4-Dihydroxy-5-halogenmethyl-tetrahydrofuran-2-yl)-1H-pyrimidin-2,4-dion der Formel IVh 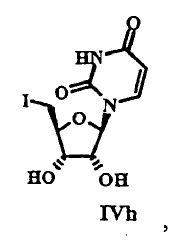
b) Kontaktieren der Verbindung der Formel IVh mit einer methanolischen Lösung aus Natriummethoxidmittel unter Herstellung des 1-(3,4-Dihydroxy-5-methylen-tetrahydrofuran-2-yl)-1H-pyrimidin-2,4-dions der Formel Vc 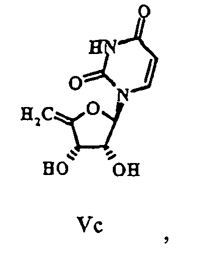
c) Kontaktieren der Verbindung der Formel Vc mit einer Lösung aus Benzyltriethylammoniumazid und Iod in Tetrahydrofuran (THF) und Acetonitril (MeCN), um das Iodazid der Formel IIe zu erhalten 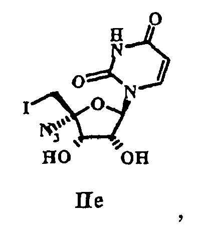
d) Kontaktieren der Verbindung der Formel IIe mit einer Lösung aus Benzoylchlorid, N-Methylmorpholin (NMM) und Acetonitril (MeCN), um die Dibenzoatverbindung der Formel IIg zu erhalten 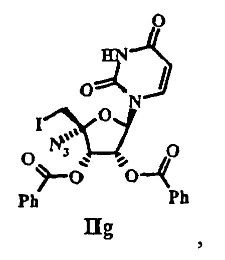
e) Kontaktieren der Verbindung der Formel IIg mit m-Chlorperbenzoesäure, m-Chlorbenzoesäure und Tetrabutylammoniumhydrogensulfat in einer Zweiphasenlösung aus Dichlormethan (DCM) und einer wässerigen Lösung aus Kaliumhydrogenphosphat, um die Verbindung der Formel IIId zu erhalten 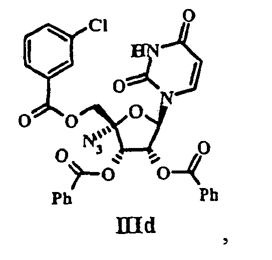
f) Kontaktieren der Verbindung der Formel IIId mit einer Lösung aus 1,2,4-Triazol, Phosphor(V)-oxidchlorid, Triethylamin (TEA) in Dichlormethan (DCM), um die Verbindung der Formel IIIe zu erhalten 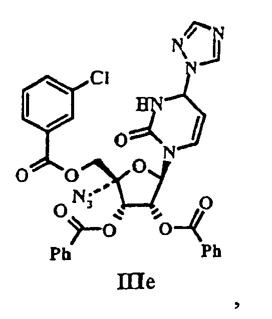
g) Kontaktieren der Verbindung der Formel IIIe mit einer Methanollösung aus Ammoniak, um die Ester zu spalten und die freie Base der Verbindung der Formel VIIIb zu erhalten 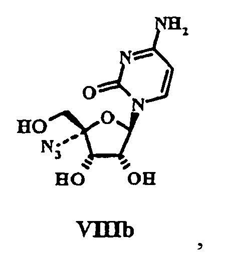
h) Kristallisieren der Verbindung der Formel VIIIb aus Isopropanol und Wasser, enthaltend H₂SO₄, um das Hemisulfatsalz der Formel VIIIc zu erhalten

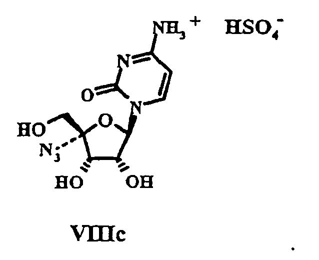

10. Verbindung gemäß der Formel Ic 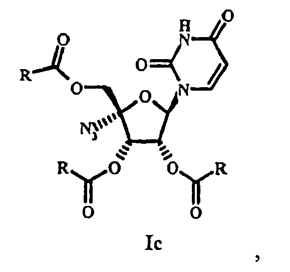 worin R Phenyl ist, gegebenenfalls substituiert mit einem bis drei Substituenten, ausgewählt aus der Gruppe, bestehend aus Halogen, C₁₋₁₀-Alkyl, C₁₋₁₀-Alkoxy, Nitro, Cyano.

## Revendications

1. Procédé de préparation d'un composé 4'-azidocytidine de formule VIII 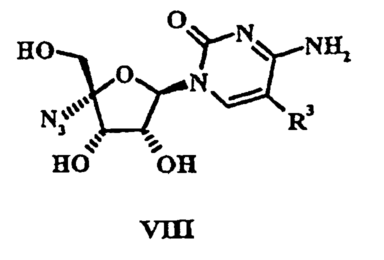 et de ses sels d'addition pharmaceutiquement acceptables,
où
R³ est H, alkyle en C₁-C₆, halogénoalkyle en C₁-C₃ ou halogéno,
**caractérisé en ce que**
dans l'étape a)
on transforme un composé nucléoside de formule VIa 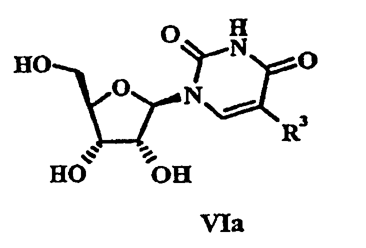 en un composé de formule II 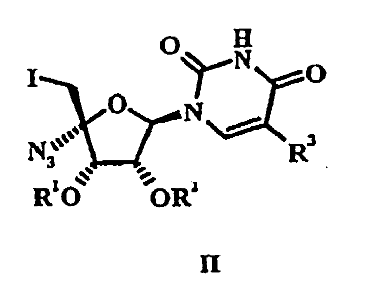 dans laquelle
R¹ est R^{1a}CO-;
R^{1a} est alkyle en C₁-C₁₀ ou phényle éventuellement substitué par 1 à 3 substituants choisis dans le groupe constitué par alkyle, alcoxy, halogéno, nitro ou cyano:
R³ est tel que défini ci-dessus,
dans l'étape b)
on met une solution de composé 5'-iodo de formule II dans un solvant approprié en contact avec un peracide, R^{2a}C(O)OOH, un acide R^{2a}C(O)OH et éventuellement un catalyseur de transfert de phase pour produire un composé 4'-azido-2',3',5'-triacylnucléoside de formule I 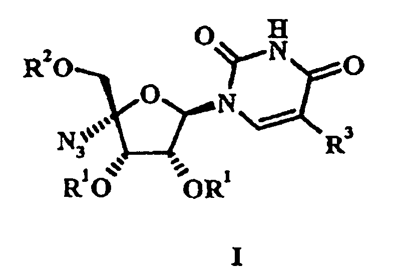 dans laquelle
R¹ est R^{1a}CO-;
R² est R^{2a}CO-;
R^{1a} et R^{2a} sont indépendamment alkyle en C₁-C₁₀ ou phényle éventuellement substitué par 1 à 3 substituants choisis dans le groupe constitué par alkyle, alcoxy, halogéno, nitro ou cyano;
R³ est tel que défini ci-dessus,
dans l'étape c)
on transforme le composé 4'-azido-2',3',5'-triacylnucléoside de formule I pour obtenir un composé 4'-azidocytidine de formule VIII et, si nécessaire, un sel d'addition pharmaceutiquement acceptable.

2. Procédé de préparation d'un 4'-azido-2',3',5'-triacylnucléoside de formule I, comprenant la mise en contact d'une solution de composé 5'-iodo de formule II dans un solvant approprié avec un peracide, R^{2a}C(O)OOH, un acide R^{2a}C(O)OH et éventuellement un catalyseur de transfert de phase pour produire un composé 4'-azido-2',3',5'-triacylnucléoside de formule I 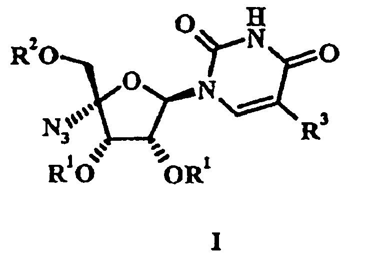 dans laquelle
R¹ est R^{1a}CO-;
R² est R^{2a}CO-;
R^{1a} et R^{2a} sont indépendamment alkyle en C₁-C₁₀ ou phényle éventuellement substitué par 1 à 3 substituants choisis dans le groupe constitué par alkyle, alcoxy, halogéno, nitro ou cyano:
R³ est H, alkyle en C₁-C₆, halogénoalkyle en C₁-C₃ ou halogéno.

3. Procédé selon la revendication 1 pour la préparation du composé 4'-azido-2',3',5'-triacylnucléoside de formule Ia, **caractérisé en ce que** ledit procédé comprend en outre la mise en contact dudit composé 4'-azido-2',3',5'-triacylnucléoside de formule IV avec une première base pour donner un composé 4'-azidonucléoside de formule Ia 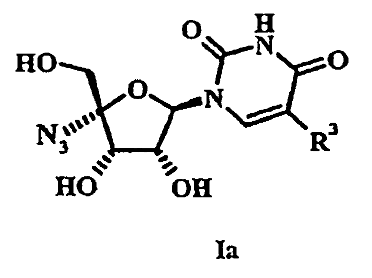 dans laquelle R₃ est tel que défini ci-dessus.

4. Procédé selon la revendication 1 pour la préparation d'un composé 5'-iodo de formule II, **caractérisé en ce que** ledit procédé comprend les étapes de
a) mise en contact du nucléoside IVa avec un agent d'halogénation pour produire un composé 5'-halogénonucléoside de formule IVb 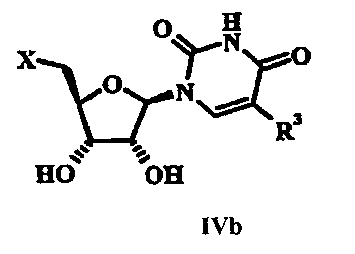 dans laquelle X est un halogène, R³ est tel que défini dans la revendication 1;
b) mise en contact du composé de formule IVb avec un agent de déshydrohalogénation pour produire un composé 5-méthylènenucléoside Va 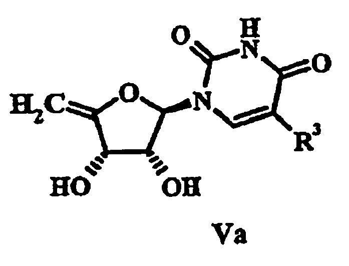 dans laquelle R³ est tel que défini ci-dessus;
c) mise en contact du composé de formule Va avec un azoture d'ammonium quaternaire et de l'iode dissous dans du tétrahydrofurane (THF) et de l'acétonitrile (MeCN) pour produire un composé iodo-azide de formule IIa 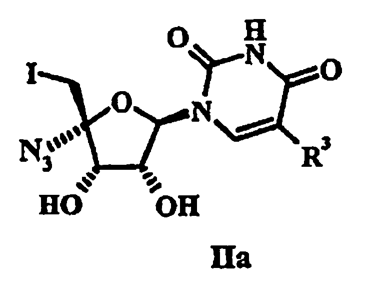 dans laquelle R³ est tel que défini ci-dessus;
d) mise en contact du composé de formule IIa avec au moins une deuxième base et un premier agent d'acylation pour donner un composé diester de formule II 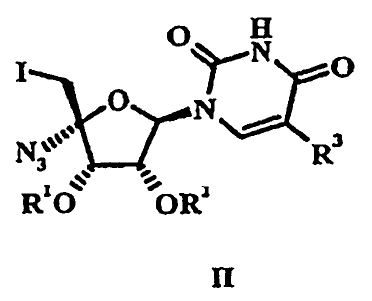
dans laquelle R¹ et R³sont tels que définis dans la revendication 1.

5. Procédé de préparation d'un composé 4'-azidocytidine de formule VIII selon la revendication 1, **caractérisé en ce que** ledit procédé comprend les étapes de
a) mise en contact d'un composé de formule I 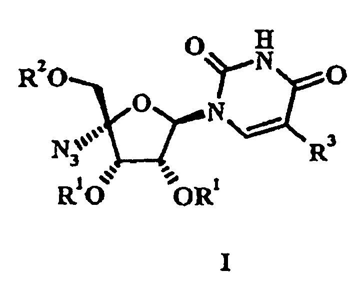 dans laquelle R¹ et R² sont définis dans la revendication 1, R³ est hydrogène, avec du 1,2,4-triazole, de l'oxychlorure de phosphore, de la triéthylamine (TEA) dans du CH₂Cl₂, pour donner un composé de triazole de formule VI 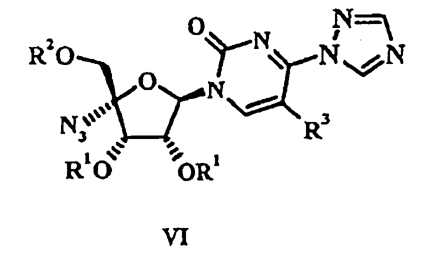 dans laquelle R¹, R² et R³ sont tels que définis ci-dessus;
b) mise en contact du composé de formule VI avec une solution d'hydroxyde d'ammonium et de tétrahydrofurane (THF) pour déplacer le triazole pour donner un composé 4'-azido-2',3',5'-triacylcytidine de formule VII 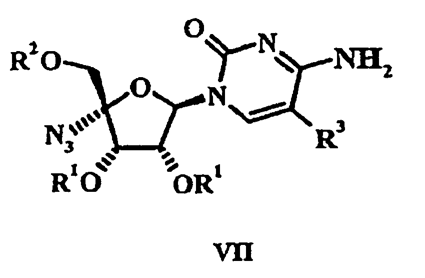 dans laquelle R¹, R² et R³ sont tels que définis ci-dessus;
c) mise en contact du composé de formule VII avec une solution d'ammoniac et d'un alcool pour dissocier les esters pour donner le composé de formule VIII 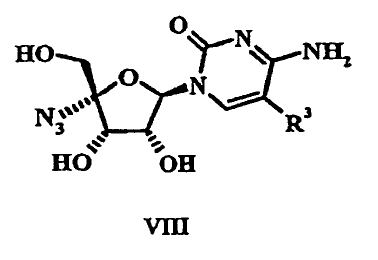
dans laquelle R¹, R² et R³ sont tels que définis ci-dessus.

6. Procédé selon la revendication 4, **caractérisé en ce que** ledit procédé comprend les étapes de:
a) mise en contact d'un composé de formule Va 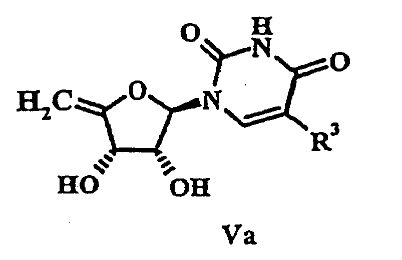 avec un deuxième agent d'acylation et éventuellement avec une base trialkylamine pour donner un composé diacylé Vb 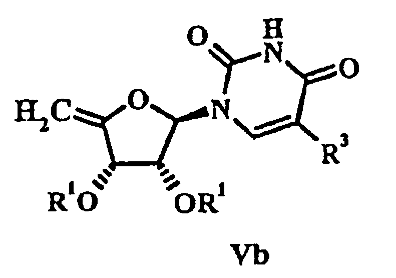 dans laquelle R¹ et R³ sont tels que définis dans la revendication 4,
et lavage d'une solution organique de Vb avec du NaHCO₃ aqueux dont on a maintenu le pH à environ 7,5; et
b) mise en contact dudit composé diacylé de formule Vb avec une solution d'ammoniac et de méthanol pour régénérer Va.

7. Procédé selon la revendication 1 pour la préparation du sel d'addition d'acide hémisulfate de 4'-azidocytidine de formule VIIIa 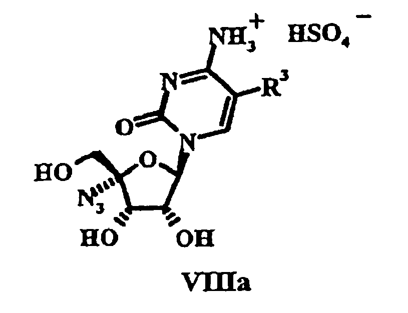 **caractérisé en ce que** ledit procédé comprend en outre la cristallisation d'un composé de formule VIII dans de l'isopropanol, de l'eau et de l'acide sulfurique.

8. Procédé selon la revendication 7 pour la préparation du sel d'addition d'acide hémisulfate de 4'-azidocytidine de formule VIIIa, dans lequel R³ est hydrogène; R¹ est PhCO; R² est R^{2a}CO où R^{2a} est phényle éventuellement substitué; ledit catalyseur de transfert de phase est un hydrogénosulfate de tétraalkylammonium, ledit solvant est un mélange d'un tampon aqueux et d'un solvant organique non polaire, ledit agent d'iodation est constitué de triphénylphosphine (TPP), d'iode et d'imidazole; ledit agent de déshydrohalogénation est le méthylate de sodium ou le 1,8-diazabicyclo[4.3.0]non-5-ène (DBN); ledit azoture d'ammonium quaternaire est l'azoture de benzyl-triéthylammonium et ledit agent d'acylation est le chlorure de benzoyle, ladite première base est l'ammoniac et ladite deuxième base est la N-méthylmorpholine (NMM) et la 4-diméthylaminopyridine (DMAP) et ledit alcool est le méthanol.

9. Procédé de préparation de l'hémisulfate de 4'-azidocytidine de formule VIIIc 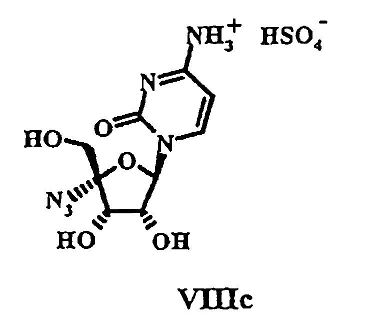 **caractérisé en ce que** ledit procédé comprend les étapes de:
a) mise en contact d'une solution dans du tétrahydrofurane (THF) d'un composé d'uridine de formule IVg 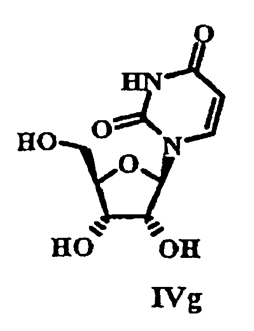 avec de la triphénylphosphine (TPP), de l'iode et de l'imidazole pour la production de 1-(3,4-dihydroxy-5-halogénométhyltétrahydrofuran-2-yl)-1H-pyrimidine-2,4-dione de formule IVh 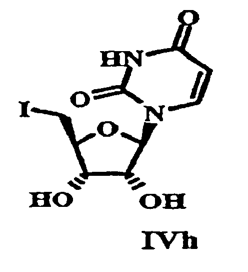
b) mise en contact du composé de formule IVh avec une solution méthanolique d'agent méthylate de sodium pour la production de 1-(3,4-dihydroxy-5-méthylènetétrahydrofuran-2-yl)-1H-pyrimidine-2,4-dione de formule Vc 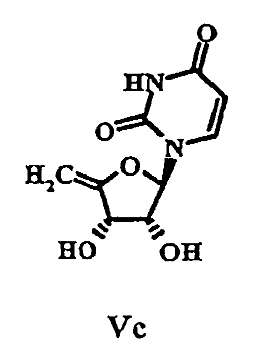
c) mise en contact du composé de formule Vc avec une solution d'azoture de benzyltriéthylammonium et d'iode dans du tétrahydrofurane (THF) et de l'acétonitrile (MeCN) pour donner un iodo-azide de formule IIe 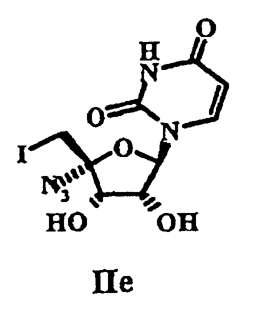
d) mise en contact du composé de formule IIe avec une solution de chlorure de benzoyle, de N-méthylmorpholine (NMM) et d'acétonitrile (MeCN) pour donner le composé dibenzoate de formule IIg: 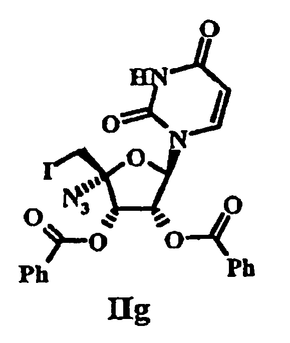
e) mise en contact du composé de formule IIg avec de l'acide m-chloroperbenzoïque, de l'acide m-chlorobenzoïque et de l'hydrogénosulfate de tétrabutylammonium dans une solution biphasique de dichlorométhane (DCM) et d'une solution aqueuse d'hydrogénophosphate de potassium pour donner le composé de formule IIId 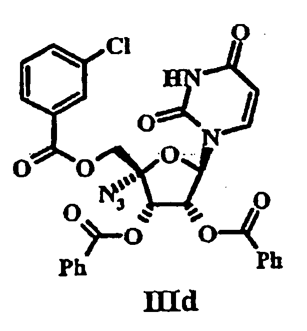
f) mise en contact du composé de formule IIId avec une solution de 1,2,4-triazole, d'oxychlorure de phosphore, de triéthylamine (TEA) dans du dichlorométhane (DCM) pour donner le composé de formule IIIe 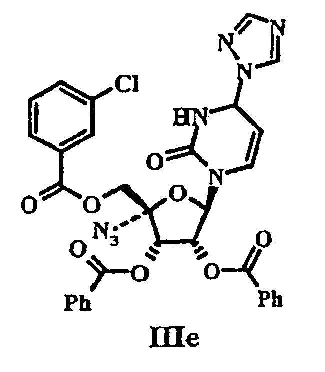
g) mise en contact du composé de formule IIIe avec une solution méthanolique d'ammoniac pour dissocier les esters et donner la base libre du composé de formule VIIIb 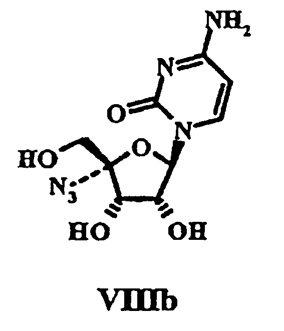
h) cristallisation du composé de formule VIIIb dans de l'isopropanol et de l'eau contenant H₂SO₄ pour donner le sel hémisulfate de formule VIIIc

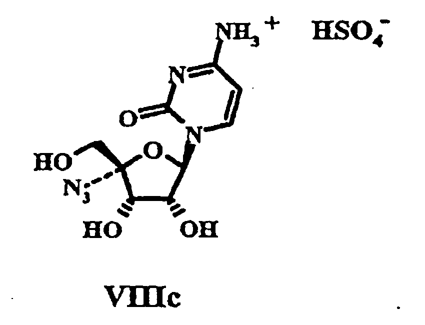

10. Composé selon la formule Ic 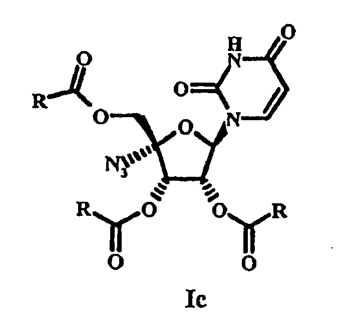 dans laquelle
R est phényle éventuellement substitué par un à trois substituants choisis dans le groupe constitué par halogéno, alkyle en C₁-C₁₀, alcoxy en C₁-C₁₀, nitro, cyano.
